# EUROPÄISCHE PATENTSCHRIFT

(11) **EP 3 458 127 B1**
(45) Veröffentlichungstag und Bekanntmachung des Hinweises auf die Patenterteilung: **01.11.2023**
(21) Anmeldenummer: 17724536.2
(22) Anmeldetag: 16.05.2017
(51) Int. Cl.: A61M 1/30, A61M 1/36

(54) **MEDIZINISCHE EINRICHTUNG MIT HYDROPHOBER FILTERMEMBRAN UND VORDERSEITIGER STÜTZSTRUKTUR HIERFÜR**
MEDICAL DEVICE HAVING AN HYDROPHOBIC FILTER MEMBRANE AND HAVING A FRONT-SIDE SUPPORTING STRUCTURE THEREFOR
DISPOSITIF MÉDICAL POURVU DE MEMBRANE FILTRANTE HYDROPHOBE ET STRUCTURE DE SUPPORT CORRESPONDANTE

(30) Priorität: 19.05.2016 DE 102016109196
(43) Veröffentlichungstag der Anmeldung: 27.03.2019
(73) Patentinhaber: Fresenius Medical Care Deutschland GmbH, 61352 Bad Homburg (DE)
(72) Erfinder: WEIS, Manfred, 66606 St. Wendel (DE); LAUER, Martin, 66606 St. Wendel (DE)
(74) Vertreter: Bobbert & Partner Patentanwälte PartmbB
(86) Internationale Anmeldenummer: PCT/EP2017/061734
(87) Internationale Veröffentlichungsnummer: WO 2017/198667

(56) Entgegenhaltungen:
- WO-A1-2010/121742
- US-A- 4 459 139

## Beschreibung

Die vorliegende Erfindung betrifft eine medizinische Einrichtung gemäß Anspruch 1.

Ein Herstellverfahren für eine medizinische Einrichtung wird weiter beschrieben.

Bekannte Blutbehandlungsvorrichtungen werden zur Blutbehandlung mit einer medizinischen Einrichtung verbunden, beispielsweise mit einer Blutkassette, in welcher Blut behandelt oder vorübergehend gespeichert wird. Derartige Blutkassetten sind aus der DE 10 2009 018 664 A1 bekannt.

US4459139 offenbart eine medizinische Einrichtung mit einem Fluidsystem für ein erstes medizinisches Fluid, einem Einrichtungskörper und einer hydrophoben Filtereinrichtung.

Aufgabe der vorliegenden Erfindung ist, eine weitere medizinische Einrichtung anzugeben.

Diese Aufgabe wird durch eine medizinische Einrichtung mit den Merkmalen des Anspruchs 1 gelöst.

Ferner wird ein nicht beanspruchtes Herstellverfahren für eine solche Einrichtung vorgeschlagen.

Erfindungsgemäß wird somit eine medizinische Einrichtung mit einem Einrichtungskörper (oder Einrichtungskorpus) vorgeschlagen.

Der Einrichtungskörper, oder ein anderer Abschnitt der medizinischen Einrichtung, weist wenigstens ein Fluidsystem für ein erstes medizinisches Fluids, insbesondere Blut, auf. Das Fluidsystem kann teilweise oder vollständig der Einrichtungskörper sein. Der Einrichtungskörper kann an der Bildung des Fluidsystems beteiligt sein. Das Fluidsystem kann jeweils einen oder mehrere Kanäle, Leitungen und/oder Fluidkammern aufweisen.

Der Einrichtungskörper, oder ein anderer Abschnitt der medizinischen Einrichtung, weist ferner wenigstens eine - vorzugsweise hydrophobe - Filtereinrichtung auf. Die Filtereinrichtung ist derart ausgestaltet und angeordnet, dass dem Fluidsystem durch die Filteroberfläche hindurch ein zweites, gasförmiges Fluid, insbesondere Luft, zuführbar ist.

Die Filtereinrichtung weist wenigstens eine Filtermembran auf. Die Filtermembran weist eine Rückseite und eine Vorderseite auf.

Die Rückseite kann jene Seite der Filtermembran sein, welche einer optionalen Fluidaufnahmekammer des Fluidsystems oder einem optionalen Abdeckelement (z. B. einer Folie) der medizinischen Einrichtung abgewandt ist.

Die Rückseite kann jene Seite der Filtermembran sein, auf welcher bei bestimmungsgemäßem Gebrauch der erfindungsgemäßen Einrichtung keine Flüssigkeit vorliegt.

Die Vorderseite kann jene Seite der Filtermembran sein, welche einer optionalen Fluidaufnahmekammer des Fluidsystems oder einem optionalen Abdeckelement (z. B. einer Folie) der medizinischen Einrichtung zugewandt ist.

Die Vorderseite kann jene Seite der Filtermembran sein, auf welcher bei bestimmungsgemäßem Gebrauch der erfindungsgemäßen Einrichtung eine Flüssigkeit vorliegt.

Die Filtermembran ist mittels ihrer Rückseite - direkt oder indirekt - oder an oder auf ihrer Rückseite mit dem Einrichtungskörper, beispielsweise mit der optionalen Fluidaufnahmekammer, oder einer Wandung hiervon, verschweißt. Hierdurch ist ein Schweißabschnitt, ein Schweißnahtabschnitt oder eine Schweißverbindung ausgestaltet. Der verschweißte Abschnitt der Filtermembran, insbesondere seine Rückseite, kann als Schweißabschnitt, Schweißnahtabschnitt oder als Schweißverbindung verstanden werden.

Die Filtereinrichtung weist eine auf der Vorderseite der Filtermembran oder vor der Filtermembran angeordnete Stützstruktur auf oder wird durch diese begrenzt. Da diese Stützstruktur an der Vorderseite der Filtermembran liegt, vor der Vorderseite liegt oder der Vorderseite zugewandt ist, wird sie hierin auch als vordere (alternativ: vorderseitige) Stützstruktur bezeichnet.

Die vordere Stützstruktur ist vorzugsweise zum Stützen der Filtermembran angeordnet oder kann in einigen Gebrauchssituationen diesem Zweck dienen. Ein Stützen kann ein Begrenzen, Halten, Kontaktieren, Berühren oder dergleichen sein. Ein Stützen kann ein Verhindern einer unzulässigen Ausbeulung der Filtermembran bei Aufbringen von Druck auf die Rückseite der Filtermembran sein.

Eine Ausbeulung kann dadurch entstehen, dass ein Druckabfall über die Filtermembran generiert wird oder auftritt. Dies kann durch ein Absenken des Drucks auf der Vorderseite oder durch ein Erhöhen des Drucks auf der Rückseite der Filtermembran erreicht werden. Das Filterelement kann ein Verbindungselement aufweisen zum Verbinden mit einer Leitung einer Blutbehandlungsvorrichtung, in der mittels einer Pumpe ein Überdruck generierbar ist. Bei dieser Pumpe kann es sich um einen Kompressor oder ein Überdruckreservoir handeln. Mittels dieser Pumpe kann ein Flüssigkeitsniveau in einer mit der Filtermembran in Kontakt stehenden Flüssigkeitskammer absenkbar sein.

Die Verwendung von "vorderer" heißt nicht, dass es eine "hintere" Stützstruktur geben muss, obgleich dies in einigen, beispielhaften Ausführungsformen der Fall sein kann.

Die vordere Stützstruktur weist einen ersten Auflageabschnitt auf und steht mit diesem mit dem Schweißabschnitt in Kontakt.

Ein nicht beanspruchtes Verfahren zum Herstellen einer medizinischen Einrichtung, insbesondere einer erfindungsgemäßen medizinischen Einrichtung, wird ferner vorgeschlagen.

Das Verfahren dient dem Herstellen einer medizinischen Einrichtung, insbesondere einer erfindungsgemäßen Einrichtung. Es umfasst das Bereitstellen eines Einrichtungskörpers einer medizinischen Einrichtung mit einem Fluidsystem für ein erstes Fluid.

Es umfasst ferner ein Bereitstellen einer Filtereinrichtung mit einer Filteroberfläche, durch welche ein zweites, gasförmiges Fluid, insbesondere Luft, durchführbar ist. Die Filtereinrichtung weist wenigstens eine Filtermembran auf; wobei die Filtermembran eine Rückseite und eine Vorderseite hat und wobei die Filtermembran mit ihrer Rückseite mit einem Abschnitt des Einrichtungskörpers entlang eines Schweißabschnitts verschweißt ist.

Weiter umfasst das Verfahren das Bereitstellen einer Stützstruktur oder vorderen Stützstruktur.

Die vordere Stützstruktur ist auf der Vorderseite der Filtermembran am Einrichtungskörper angeordnet. Dies geschieht derart, dass die vordere Stützstruktur mittels eines ersten Auflageabschnitts der vorderen Stützstruktur mit dem Schweißabschnitt in Kontakt steht.

Schließlich wird die vordere Stützstruktur mit der Vorderseite der Filtermembran verschweißt. Letzteres kann unter Erweichen von Material der vorderen Stützstruktur erfolgen.

Bei allen Ausführungen hierin ist der Gebrauch des Ausdrucks "kann sein" bzw. "kann haben" usw. synonym zu "ist vorzugsweise" bzw. "hat vorzugsweise" usw. zu verstehen und soll eine erfindungsgemäße Ausführungsform erläutern.

Erfindungsgemäße Ausführungsformen können eines oder mehrere der vorstehend oder im Folgenden genannten Merkmale aufweisen. Dabei können die hierin genannten Merkmale in beliebiger Kombinationen Gegenstand von erfindungsgemäßen Ausführungsformen sein, sofern der Fachmann eine konkrete Kombination nicht als technisch unmöglich erkennt. Erfindungsgemäße Ausführungsformen sind ferner Gegenstand der Unteransprüche.

Wann immer hierin Zahlenworte genannt werden, so versteht der Fachmann diese als Angabe einer zahlenmäßig unteren Grenze. Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

Die Angaben "oben" und "unten" sind hierin bei Zweifel des Fachmanns als absolute oder relative Raumangaben zu verstehen, welche sich auf die Ausrichtung des betreffenden Bauteils während seines üblichen Gebrauchs beziehen.

Die Angaben "vordere" und "hintere" usw. sind hierin bei Zweifel des Fachmanns als absolute oder relative Raumangaben zu verstehen, welche sich auf die medizinische Einrichtung beziehen. Weist die medizinische Einrichtung ein Abdeckelement auf, so liegt dieses auf der vorderen Seite oder Vorderseite der medizinischen Einrichtung.

Unter einem "Kontakt" kann hierin stets beispielsweise ein Berühren, ein Kraftschluss, ein Formschluss, ein Stoffschluss (z. B. durch Verschweißen miteinander) oder ein gegenseitiges Verpressen zwischen den in Frage bzw. "in Kontakt" stehenden Komponenten verstanden werden. Ein Kontakt kann ein direkter Kontakt (z. B. ein direktes Berühren) oder ein indirekter Kontakt (z. B. über eine Zwischenschicht hinweg; die Zwischenschicht kann eine Membran sein) sein.

In einigen erfindungsgemäßen, beispielhaften Ausführungsformen ist die erfindungsgemäße medizinische Einrichtung ein Schlauchsystem, ein Schlauchset, eine Blutkassette oder jeweils ein Teil hiervon.

Der Begriff "Fluidaufnahmekammer", wie er hierin verwendet wird, bezeichnet beispielsweise eine Kammer, oder einen Teil hiervon, oder einen Behälter, oder einen Teil hiervon, welche/r ein Inneres oder einen Innenraum aufweist, der zum vollständigen oder teilweise Befüllen mit Fluiden und Aufnehmen derselben geeignet und bestimmungsgemäß vorgesehen ist.

In einigen erfindungsgemäßen, beispielhaften Ausführungsformen weist die Fluidaufnahmekammer genau zwei Fluidzu- oder -ausgänge auf. Eine der beiden führt durch die Filtermembran hindurch und dient dem Zuführen und/oder Abführen von Gas, vorzugsweise nur von Gas, nicht auch von Flüssigkeit. Der andere ist der Fluidanschluss zum Zuführen und/oder Abführen von Flüssigkeit, etwa dem ersten Fluid.

Ein "erstes Fluid" im Sinne der vorliegenden Erfindung schließt jede medizinische Flüssigkeit und/oder jedes medizinische Gas sowie beliebige Kombinationen ein, welche(s) zum Einbringen in eine erfindungsgemäße Aufnahmeeinrichtung angedacht bzw. vorgesehen ist. Vorzugsweise handelt es sich bei dem ersten Fluid um Blut.

Der Begriff "erstes Fluid" wird deshalb gleichbedeutend mit dem Begriff "medizinisches Fluid" verwendet.

Ein "zweites Fluid" ist ein gasförmiges Fluid, oder weist ein Gas auf, vorzugsweise Luft.

Der Begriff "Filtereinrichtung" oder "hydrophobe Filtereinrichtung", wie er hierin gleichbedeutend verwendet wird, bezeichnet beispielsweise eine Einrichtung, welche dazu ausgelegt und vorgesehen ist, eine Filterwirkung auf Fluide, welche durch die Fluidaufnahmekammer hindurchzuführen sind, auszuüben. Alternativ bezeichnet eine Filtereinrichtung i. S. d. Erfindung eine Membran ohne Filterwirkung.

Die Filterwirkung kann z. B. eine Reinigung der der Fluidaufnahmekammer zuzuführenden Fluide, insbesondere zum Beispiel das Zurückhalten von Feststoffen, Kleinstlebewesen, wie Viren oder Bakterien, und dergleichen umfassen.

Die Filterwirkung kann auch umfassen, dass der Filter durchlässig für Gas, nicht jedoch für eine Flüssigkeit, insbesondere Wasser oder Blut, ist. Dadurch kann es möglich sein, ein Eindringen der Flüssigkeit in einen Bereich jenseits der Filtermembran zu verhindern. Ist beispielsweise die durch die Filtermembran begrenzte Fluidkammer durch eine Öffnung mit der Maschine verbunden, kann verhindert werden, dass die Maschine kontaminiert wird, wenn die Flüssigkeit in Richtung zur Filtermembran vordringt.

In einigen, beispielhaften erfindungsgemäßen Ausführungsformen weist die vordere Stützstruktur einen zweiten Auflageabschnitt auf. Dabei ist die vordere Stützstruktur angeordnet, um mittels des zweiten Auflageabschnitts mit einem Stützabschnitt des Einrichtungskörpers in Kontakt zu stehen, und/oder steht mit diesem in Kontakt.

In einigen, beispielhaften erfindungsgemäßen Ausführungsformen ist der zweite Auflageabschnitt mit dem Stützabschnitt des Einrichtungskörpers verschweißt.

In einigen, beispielhaften erfindungsgemäßen Ausführungsformen ist der zweite Auflageabschnitt mit dem Stützabschnitt des Einrichtungskörpers verklebt.

In einigen, beispielhaften erfindungsgemäßen Ausführungsformen sind der Schweißabschnitt und/oder der erste Auflageabschnitt umlaufende oder geschlossene Abschnitte oder Ringbereiche.

In einigen, beispielhaften erfindungsgemäßen Ausführungsformen ist der zweite Auflageabschnitt mit dem Stützabschnitt des Einrichtungskörpers verklemmt.

Hierzu kann eine Klemmeinrichtung vorgesehen sein.

Die Klemmeinrichtung kann als Bördelung oder Bördelkante ausgestaltet sein oder eine solche aufweisen.

Die Klemmeinrichtung, Bördelung oder Bördelkante können umlaufende oder geschlossene Abschnitte sein.

In einigen, beispielhaften erfindungsgemäßen Ausführungsformen ist der erste Auflageabschnitt der vorderen Stützstruktur aus einem weicheren Material als andere Abschnitte oder als alle anderen Abschnitte der vorderen Stützstruktur gefertigt oder weist ein weicheres Material als jene Abschnitte auf.

Als (relativ zu anderen Abschnitten gesehen) "weicheres" Material kommt Silikon in Frage. Dies ist vergleichsweise teuer, weshalb es sich anbietet, beispielsweise nur den ersten Auflageabschnitt hieraus zu fertigen. Für andere Abschnitte der vorderen Stützstruktur, für welche die besonderen Eigenschaften von Silikon nicht erforderlich sind oder gar nachteilig sein können (etwa die hohe Nachgiebigkeit von aus Silikon gefertigten Komponenten, welche der Idee des Stützens zuwiderläuft), kann ein - verglichen mit Silikon oder verglichen mit dem Material des ersten Auflageabschnitts - steiferes oder härteres Material ausgewählt sein.

In einigen, beispielhaften erfindungsgemäßen Ausführungsformen ist der erste Auflageabschnitt der vorderen Stützstruktur dauerhaft aus einem weicheren Material, in anderen ist das betreffende Material nur vorübergehend, etwa bei der Fertigung (durch Schweißen, z. B.) weicher und verhärtet sich optional anschließend wieder.

In einigen, beispielhaften erfindungsgemäßen Ausführungsformen ist die vordere Stützstruktur aus genau einem Material gefertigt oder besteht aus genau einem Material. In einigen, beispielhaften erfindungsgemäßen Ausführungsformen steht der erste Auflageabschnitt der vorderen Stützstruktur mit dem Schweißnahtabschnitt des Einrichtungskörpers in - vorzugsweise direktem - Kontakt. Vorzugsweise steht der Schweißnahtabschnitt ausschließlich mit dem ersten Auflageabschnitt in Verbindung.

Erfindungsgemäß überragt der erste Auflageabschnitt der vorderen Stützstruktur den Schweißabschnitt des Einrichtungskörpers in wenigstens einer Richtung oder auf wenigstens einer Seite des Schweißabschnitts oder steht über den Schweißabschnitt über. Die Richtung kann parallel zur Haupterstreckungsrichtung der vorderen Stützstruktur oder der Filtermembran verlaufen. Ein Überragen oder Überstehen erfolgt erfindungsgemäß in Richtung auf einen inneren oder zentralen Abschnitt der Filtermembran oder der vorderen Stützstruktur und/oder in Richtung eines Bereichs der Filtermembran, der im Gebrauch mit Druck oder am stärksten mit Druck beaufschlagt wird.

In einigen, beispielhaften erfindungsgemäßen Ausführungsformen ist der erste Auflageabschnitt der vorderen Stützstruktur lösbar von weiteren Abschnitten der vorderen Stützstruktur mit den weiteren Abschnitten verbunden. Die Verbindung kann durch ein Einstecken erzielt sein.

In einigen, beispielhaften erfindungsgemäßen Ausführungsformen ist die vordere Stützstruktur ein flächiges oder planes Gitter oder weist ein solches auf.

In einigen, beispielhaften erfindungsgemäßen Ausführungsformen weist die vordere Stützstruktur Öffnungen auf.

In einigen, beispielhaften erfindungsgemäßen Ausführungsformen unterscheidet sich der erste Auflageabschnitt der vorderen Stützstruktur farblich von weiteren Abschnitten der vorderen Stützstruktur.

So kann der erste Auflageabschnitt z. B. blau oder einen anderen vergleichsweise dunklen Ton haben, während die vordere Stützstruktur grau oder weiß oder transparent ist. Auf diese Weise kann der die medizinische Einrichtung fertigende Mitarbeiter oder der die medizinische Einrichtung später zur Behandlung des Patienten einsetzende Arzt oder Mitarbeiter auf Station auf einen Blick überprüfen, ob die oftmals separat vom Einrichtungskörper gefertigte vordere Stützstruktur tatsächlich in den Einrichtungskörper eingelegt wurde. Dies erleichtert die Endkontrolle nach Beendigung der Herstellung. Zudem kann es zur Sicherheit des Patienten beitragen, indem fehlerhafte medizinische Einrichtungen selbst am Patientenbett noch frühzeitig erkannt werden können.

In einigen, beispielhaften erfindungsgemäßen Ausführungsformen weist die vordere Stützstruktur Öffnungen auf. Der Durchmesser oder die Breite der Öffnungen beträgt maximal 0,7 mm, vorzugsweise maximal 0,5 mm. Diese Maße haben sich als bevorzugt erwiesen, um einen Kompromiss zwischen Durchlassfläche einerseits und Abstützung der Filtermembran andererseits zu gewährleisten. Sind die Öffnungen größer als hierin angegeben, so kann die Filtermembran bei Druck zu sehr in die Öffnungen hineingedrückt werden. Dies kann eine Durchlässigkeit vermindern. Ferner kann die Filtermembran unerwünscht stark mechanisch belastet werden. Bei den vorgeschlagenen Öffnungsgrößen kann die Filtermembran vorteilhaft mit mindestens 3 bar beaufschlagt werden.

In einigen, beispielhaften erfindungsgemäßen Ausführungsformen weist die vordere Stützstruktur eine zweite Filtermembran auf oder besteht hieraus.

In einigen, beispielhaften erfindungsgemäßen Ausführungsformen ist der erste Auflageabschnitt der vorderen Stützstruktur ein während der Herstellung der Einrichtung durch Aufschmelzen oder vorübergehendem Verflüssigen von Material entstandener Abschnitt oder weist einen solchen auf. Dieser aufgeschmolzene Abschnitt kann z. B. als ein flächig verlaufender Materialabschnitt erkennbar sein, der sich von den verglichen hiermit klar definierten, nicht aufgeschmolzenen und/oder optisch erkennbar auf andere Weise gefertigten oder behandelten, übrigen Strukturen der vorderen Stützstruktur abgrenzt.

In einigen, beispielhaften erfindungsgemäßen Ausführungsformen weist die vordere Stützstruktur in ihrem dem Schweißnahtabschnitt zugewandten Bereich eine profilierte oder laminierte Struktur auf. Die Profilierung oder Laminierung kann dabei parallel oder schräg zur Haupterstreckungsebene der Stützstruktur verlaufende Kanäle ergeben. Diese Kombination zwischen erhabenen Strukturen und Kanälen, also die Profilierung, kann bewirken, dass bei einem Aufschmelzen der Struktur zum Befestigen der Filtermembran an der vorderen Stützstruktur aufgeschmolzenes Material nicht oder nur in geringer Menge in den zentralen Bereich der Filtermembran eindringt, sondern sich überwiegen in die Kanalbereiche ausdehnt. Dies kann die Öffnungen der vorderen Stützstruktur und die Filtermembran vor einem Verkleben durch aufgeschmolzenes Material schützen.

Die Filtereinrichtung ist in einigen, beispielhaften erfindungsgemäßen Ausführungsformen derart angeordnet, dass ein Normalenvektor auf die Filteroberfläche der optional hydrophoben Filtereinrichtung bei bestimmungsgemäßem Gebrauch der medizinischen Einrichtung und mehr oder weniger mit erstem Fluid befüllter Fluidaufnahmekammer nicht parallel zu einem Normalenvektor auf die Ebene des Fluidpegels bzw. des Fluidspiegels des in der Fluidaufnahmekammer vorhandenen ersten Fluids verläuft. Die beiden Normalenvektoren liegen, anders ausgedrückt, in diesen erfindungsgemäßen Ausführungsformen nicht in einer Ebene.

Der Begriff "Normalenvektor", wie er hierin in Bezug auf die Filteroberfläche verwendet wird, bezeichnet in einigen, beispielhaften erfindungsgemäßen Ausführungsformen einen Normalenvektor zu einem beliebigen Oberflächenabschnitt der Filtereinrichtung. Der Normalenvektor auf die Filteroberfläche kann eine Senkrechte zu einer Haupterstreckungsebene der Filtereinrichtung, besonders bevorzugt eine Senkrechte zu einer Oberfläche eines Hauptabschnitts der Filtereinrichtung, ganz besonders bevorzugt eine Senkrechte zu einem mittleren Abschnitt der Filtereinrichtung oder eine Senkrechte zu einem filternden Abschnitt der Filtereinrichtung oder der nicht-filternden Membran sein. Ein solcher Normalenvektor auf die Filteroberfläche kann ein senkrechtes Lot oder eine Senkrechte auf einen der zuvor genannten Abschnitte darstellen.

Der Begriff "Normalenvektor auf die Ebene des Fluidspiegels", wie er hierin verwendet wird, bezeichnet - in Anlehnung an die vorstehend angegebene Definition für den Normalenvektor auf die Filteroberfläche - einen Normalenvektor zu einem beliebigen Abschnitt bzw. Bereich der Ebene des Fluidspiegels.

Der Begriff "Fluidspiegel" bezieht sich erfindungsgemäß auf einen Fluidspiegel oder - pegel (diese beiden Begriffe werden nachfolgend synonym zueinander verwendet) eines zum Gebrauch der medizinischen Einrichtung in die Fluidaufnahmekammer aufgenommenen ersten Fluids.

Bei Schwapp- und/oder Strömungsbewegungen, beispielsweise Rotations- und/oder Wellenbewegungen der sich in der Fluidaufnahmekammer befindenden Fluide kann es schwierig sein, einen Fluidspiegel oder eine Senkrechte hierauf zu bestimmen. Daher wird unter einem Fluidspiegel erfindungsgemäß bevorzugt ein unter Berücksichtigung aller Schwapp- und/oder Strömungsbewegungen der sich in der Fluidaufnahmekammer befindenden Fluide gemittelter Durchschnittspegel oder -spiegel verstanden.

Der Fluidspiegel kann vorzugsweise in einem Strömungs-Ruhezustand des Fluids bestimmt werden. Es ist unerheblich, ob ein solcher Ruhezustand im Gebrauch der medizinischen Einrichtung erreicht wird. Es genügt für die vorliegenden Zwecke, einen solchen anzunehmen oder zu approximieren.

In einigen, beispielhaften erfindungsgemäßen Ausführungsformen steht der Normalenvektor auf die Filteroberfläche im Wesentlichen senkrecht zum Normalenvektor auf die Ebene des Fluidspiegels.

Der Begriff "im Wesentlichen senkrecht", wie er hierin verwendet wird, umfasst Abweichungen von der Rechtwinkeligkeit, die z.B. daher rühren, dass die erfindungsgemäße medizinische Einrichtung, welche die Einrichtung aufweist, im Gebrauch eine geringe Neigung, z.B. bis zu +/- 15°, aufweist, während bei einer solchen Neigung der Filtereinrichtung der Fluid- oder Flüssigkeitspegel in der medizinischen Einrichtung trotzdem horizontal bleibt.

In einigen, beispielhaften erfindungsgemäßen Ausführungsformen hat ein Normalenvektor auf die Filteroberfläche keinen Schnittpunkt mit einem Fluidspiegel - wie oben definiert - des während des Gebrauchs in der Fluidaufnahmekammer vorhandenen ersten Fluids.

In einigen, beispielhaften erfindungsgemäßen Ausführungsformen weist die medizinische Einrichtung wenigstens eine Fluidzuführungskammer zum Zuführen des zweiten Fluids auf.

Der Begriff "Fluidzuführungskammer", wie er hierin verwendet wird, bezeichnet eine Kammer oder einen Behälter, welche/r ein Inneres oder einen Innenraum aufweist, der zum Aufnehmen eines zweiten Fluids und Zuführen desselben in die Fluidaufnahmekammer geeignet und bestimmt ist.

Die Fluidzuführungskammer kann in Form einer Spritzgusskammer hergestellt sein. Die Fluidzuführungskammer kann eine Einmal-Fluidzuführungskammer sein.

Die Fluidzuführungskammer kann in wenigstens einem Abschnitt derselben mit der Fluidaufnahmekammer verbunden sein.

Sie kann stoffschlüssig mit der Fluidaufnahmekammer verbunden oder in diese integriert sein.

Die Fluidzuführungskammer kann beispielsweise während der Herstellung der Fluidaufnahmekammer ausgestaltet worden sein.

Die Fluidzuführungskammer kann durch wenigstens eine Abgrenzung oder eine Teilwand von der Fluidaufnahmekammer abgetrennt sein.

In einigen, beispielhaften erfindungsgemäßen Ausführungsformen ist die Filtereinrichtung in einem Inneren der Fluidzuführungskammer angeordnet.

Die Fluidzuführungskammer und die Fluidaufnahmekammer stehen bevorzugt lediglich über die Filtereinrichtung miteinander in Fluidkommunikation.

Bevorzugt wird dabei das zweite Fluid im Gebrauch der medizinischen Einrichtung aus der Fluidzuführungskammer durch die Filtereinrichtung in die Fluidaufnahmekammer eingebracht bzw. dieser zugeführt.

Ganz besonders bevorzugt ist die Filtereinrichtung derart ausgestaltet und vorgesehen, dass ein sich in der Fluidaufnahmekammer befindendes erstes Fluid und insbesondere ein flüssiges Fluid nicht durch die Filtereinrichtung in die Fluidzuführungskammer gelangen kann.

In einigen, beispielhaften erfindungsgemäßen Ausführungsformen kann das zweite Fluid über einen Fluidkonnektor, welcher mit der Fluidzuführungskammer verbindbar oder verbunden ist, in die Fluidzuführungskammer eingebracht, eingeströmt, eingeleitet oder dergleichen werden.

Der Fluidkonnektor kann ein hülsenförmiges Gebilde sein. Er kann aus einem Stück mit einer Wandung bzw. Seitenwand der Fluidzuführungskammer hergestellt sein. Er kann auf bzw. an einer Außenseite der Wandung oder Seitenwand der Fluidzuführungskammer vorgesehen sein. Er kann innerhalb eines Bereichs der in der Fluidzuführungskammer angeordneten Filtereinrichtung vorgesehen sein. Er kann direkt mit der Filtereinrichtung verbunden bzw. an diese angeschlossen sein.

Der Fluidkonnektor kann über eine oder mehrere Verbindungsbohrungen mit einer Innenseite der Wandung oder Seitenwand der Fluidzuführungskammer verbunden sein.

Der Fluidkonnektor kann angeordnet sein, um unmittelbar ein Fluid von einem äußeren der medizinischen Einrichtung in ein Inneres einzuführen.

In einigen, beispielhaften erfindungsgemäßen Ausführungsformen kann die Fluidaufnahmekammer beispielsweise in Form einer Spritzgusskammer hergestellt sein.

Die Fluidaufnahmekammer kann eine Einmal-Fluidaufnahmekammer sein.

Die Fluidaufnahmekammer kann eine Fluidverbindung zu einem Äußeren der Kammer aufweisen. Sie kann zwei oder mehr Fluidverbindungen zum Äußeren der Kammer aufweisen.

Die Fluidaufnahmekammer kann eine oder genau eine Fluidverbindung allein zu einem Inneren der Kammer aufweisen. Sie kann zwei oder mehr Fluidverbindungen zum Inneren der Kammer aufweisen.

Die Fluidaufnahmekammer ist mittels der Filtermembran bevorzugt vom Fluidkonnektor der Fluidzuführungskammer und/oder von einem Äußeren der medizinischen Einrichtung entkoppelt, was eine Flüssigkeitsverbindung betrifft.

Die Filtermembran kann eine beliebige geeignete Form aufweisen. Sie kann beispielsweise rund, eckig, insbesondere rechteckig, elliptisch und dergleichen ausgestaltet sein.

Die Filtermembran kann aus einem Filtermembranband ausgeschnitten und/oder gegebenenfalls auf eine bestimmte Form zugeschnitten werden oder sein.

Die Filtermembran kann eine Einmal-Filtermembran sein.

Die Filtermembran kann eine hydrophobe Membran sein. Die Filtermembran kann an wenigstens einer Seite hydrophob sein.

Die Filtermembran kann aus zwei Schichten bestehen, nämlich erstens der eigentlichen Membran, die zumeist aus einem schwer schweißbaren und schwer klebbaren Werkstoff besteht, wie z.B. PTFE (Polytetrafluorethylen) oder ePTFE (expanded Polytetrafluoroethylen), und zweitens einer Schicht, die eine Drainage- und/oder Stützfunktion hat und zumeist aus Gewebe und/oder Vlies besteht oder dieses aufweist, das schweißbar und/oder klebbar ist. Die Filtermembran kann alternativ neben den zuvor genannten Schichten weitere Schichten oder Bestandteile aufweisen.

Die Filtermembran kann eine Sterilmembran sein.

Die Filtermembran kann aus einem nicht oder nur schwierig verklebbaren und/oder einem nicht oder nur schwierig verschweißbaren Werkstoff bestehen oder einen solchen aufweisen.

Die Filtermembran kann während der Verwendung der medizinischen Einrichtung, wie beispielsweise der Dauer einer Behandlung, wechselnden Drücken aus Richtung der Fluidaufnahmekammer (ausströmendes Gas, anstehende Flüssigkeit) und/oder aus Richtung einer Behandlungsvorrichtung (einströmendes Gas, anstehende Störfallflüssigkeit und/oder aus Kondensation entstehende unerwünschte Flüssigkeit) ausgesetzt sein. Zum Schutz der Filtermembran im flächigen Nutzbereich und/oder an den Stellen ihrer Abdichtung (in der Regel Verschweißung) z.B. an Wandungen der Fluidzuführungskammer vor Verformung durch diese Druckdifferenzen, welche zu einem Strukturschaden oder einem Riss in der Membran führen könnte, kann es von Vorteil sein, die Filtermembran mechanisch abzustützen.

Zum Erzielen einer solchen mechanischen Abstützung kann eine Stützstruktur eingesetzt werden. Diese ist derart ausgestaltet, dass die Stützflächen die Filtermembran nicht unzulässig gegen den gewünschten Fluiddurchtritt durch die Filtermembran, z.B. einen Nutzgasdurchtritt, abdichten.

Die Filtereinrichtung kann symmetrisch oder asymmetrisch aufgebaut sein.

In einigen, beispielhaften erfindungsgemäßen Ausführungsformen weist die Filtereinrichtung auf bzw. an beiden Seiten eine Stützstruktur auf, also sowohl an der Vorderseite als auch an der Rückseite der Filtermembran.

Zusätzlich zu der vorderen Stützstruktur kann auf der Rückseite der Filtermembran eine hierin als "hintere" Stützstruktur bezeichnete Stützstruktur vorliegen. Die beiden Stützstrukturen können getrennt voneinander vorliegen.

Eine dritte Stützstruktur kann auf der Membranschicht selbst angeordnet sein. Sie kann vorzugsweise als Vlies, Gewebe oder dergleichen ausgestaltet sein.

Die hintere Stützstruktur ist vorzugsweise im Inneren des Gehäuses des Fluidkonnektors vorgesehen. Sie kann eine Drainagestruktur aufweisen. Die hintere Stützstruktur kann eine Drainagewirkung haben.

Die hintere Stützstruktur kann kraft- und/oder form- und/oder stoffschlüssig mit der Fluidzuführungskammer verbunden sein. Bevorzugt ist die hintere Stützstruktur in einem äußeren umlaufenden Bereich kraft- und/oder form- und/oder stoffschlüssig mit der Fluidzuführungskammer oder einem anderen Abschnitt des Einrichtungskörpers verbunden.

Da die Filtermembran in der Regel aus einem nicht oder schlecht verklebbaren und/oder einem nicht oder schlecht verschweißbaren Werkstoff besteht, kann die dem Inneren der Fluidzuführungskammer zugewandte Stützstruktur in wenigstens einem äußeren Bereich, bevorzugt einem äußeren, umlaufenden Bereich, derselben mit der Fluidzuführungskammer verbunden sein.

Bevorzugter Weise ist die hintere Stützstruktur stoffschlüssig mit der Fluidzufiihrungskammer oder einem anderen Abschnitt des Einrichtungskörpers verbunden, beispielsweise verschweißt, z.B. mittels Thermoschweißen, Spiegelschweißen, Ultraschallschweißen oder Laserschweißen. Die hintere Stützstruktur kann thermisch mit der Fluidzuführungskammer bzw. einem Verbindungsbereich derselben verschweißt sein.

Zu diesem Zweck ist die hintere Stützstruktur - bevorzugt in einem äußeren Bereich derselben - aus einem höher schmelzenden Werkstoff als der Verbindungsbereich der Fluidzuführungskammer hergestellt.

Beim Verschweißen der hinteren Stützstruktur mit der Fluidzuführungskammer bzw. einem Verbindungsbereich derselben kann der durch Erwärmen verflüssigte Werkstoff der Fluidzuführungskammer in eine poröse Struktur der hinteren Stützstruktur eindringen. Der flüssige Werkstoff kann bis zur Filtermembran eindringen. Auf diese Weise kann vorteilhaft eine unlösbare Verbindung zwischen der Fluidzuführungskammer, der hinteren Stützstruktur und der Filtermembran gebildet werden. Gleichzeitig kann die Filtermembran besonders seitlich vorteilhaft abgedichtet werden.

Die hintere Stützstruktur ist vorzugsweise ein dünnwandiges Spritzgussteil. Sie weist bevorzugt auf der der Membranschicht zugewandten Seite oder beiden eine Drainagestruktur auf.

Die hintere und/oder die vordere Stützstruktur können mittels Spritzguss hergestellt sein.

Beispielsweise kann die hintere Stützstruktur in eine spritzgusstechnisch erzeugte Wandung der Fluidzuführungskammer integriert sein.

Die vordere Stützstruktur kann im Wesentlichen parallel zur Filtermembran angeordnet sein.

Die vordere Stützstruktur kann kraftfrei an der Filtermembran anliegend und/oder mit geringem Spiel an der dem Inneren der Fluidzuführungskammer abgewandten Seite der Filtermembran vorgesehen sein oder mit der Filtermembran in Kontakt stehen.

Die vordere Stützstruktur kann aus dem gleichen Werkstoff wie die Fluidzuführungskammer hergestellt sein oder eine solchen aufweisen.

Die vordere Stützstruktur kann ein separates Element bilden bzw. als solches hergestellt sein. Beispielsweise kann die vordere Stützstruktur als dünnwandiges Spritzgussbauteil gefertigt sein.

In einigen, beispielhaften erfindungsgemäßen Ausführungsformen überdeckt die vordere Stützstruktur die Filtermembran im Wesentlichen vollständig.

Die vordere Stützstruktur kann in ihrer Fläche und/oder Berandung nach Außen durch eine Ringzone oder einen Ringbereich ohne Drainagewirkung abgeschlossen sein.

Die Begriffe "Ringzone" oder "Ringbereich" bezeichnen in einigen, beispielhaften erfindungsgemäßen Ausführungsformen einen äußeren Bereich oder äußeren Rand bzw. Außenrand der vorderen Stützstruktur. Der Wortteil "Ring-" soll die Erfindung dabei jedoch nicht auf eine kreisförmige Ausgestaltung der Zone oder des Bereichs einschränken. Vielmehr soll er einen umlaufenden Bereich oder eine umlaufende Zone beschreiben, welche jedoch auch in jeder beliebigen anderen geeigneten Form ausgestaltet sein kann, beispielsweise in Form eines Rechtecks, einer Ellipse und dergleichen.

Eine Außenberandung dieser Ringzone oder dieses Ringbereichs kann im Wesentlichen den Außenabmessungen der Filtermembran entsprechen.

Eine Innenberandung dieser Ringzone oder dieses Ringbereichs kann im Wesentlichen dem nach der Befestigung der Filtermembran an der Ringzone oder dem Ringbereich verbleibenden filterwirksamen Filtermembranareal entsprechen.

Die Filtermembran kann gasdicht mit der vorderen Stützstruktur verbunden sein. Sie kann mit einem Wandungsmaterial der Ringzone oder des Ringbereichs verbunden sein. Beispielsweise kann die Filtermembran in einem äußeren, bevorzugt umlaufenden, Bereich derselben mit der vorderen Stützstruktur bzw. einem äußeren Ringbereich derselben verbunden sein.

Die Filtermembran kann stoffschlüssig mit dem Wandungsmaterial verbunden sein. Sie kann beispielsweise mit dem Wandungsmaterial verklebt oder verschweißt sein. Als Schweißverfahren kommen u. a. Thermoschweißen, Spiegelschweißen, Ultraschallschweißen oder Laserschweißen in Frage.

Die Filtermembran kann entsprechend sowohl mit der hinteren Stützstruktur als auch mit der vorderen Stützstruktur verbunden oder integriert sein.

Die vordere Stützstruktur kann ein dünnwandiges Spritzgussteil bzw. -element sein, welches mittels Verschweißen und/oder Verkleben mit der Filtermembran und/oder dem Einrichtungskörper verbindbar ist.

Die vordere Stützstruktur kann u. a. mittels Thermoschweißen, Spiegelschweißen, Ultraschallschweißen oder Laserschweißen verschweißt sein.

Ein wenigstens drei Stützstrukturen aufweisendes Konstrukt kann beispielhaft wie folgt gebildet werden:
Auf die Filtermembran wird die dritte Stützschicht aufgebracht. Die dritte Stützschicht, z.B. ein schweißbares Vlies, wird bei der Montage auf der Seite des Vlieses auf die hintere Stützstruktur gelegt. Die dritte Stützschicht wird am Umfang der Filtermembranschicht, d.h. vorzugsweise in dem Bereich, welcher außerhalb der hinteren Stützstruktur liegt, umlaufend dicht mit der hinteren Stützstruktur verschweißt und/oder verklebt. Auf diese Weise kann vorteilhaft eine Dichtfunktion zwischen der dritten Stützstruktur und der hinteren Stützstruktur erreicht werden.

Die vordere Stützstruktur, z.B. ein dünnwandiges Spritzgussteil, wird wie ein Deckel mit ihrer Stützstruktur auf die an der hinteren Stützstruktur optional angeschweißte Filtermembran gelegt. Sie wird in einer äußeren Ringzone außerhalb der Filtermembranschicht mit dem Gehäuse (Spritzgussgehäuse) des Fluidkonnektors verschweißt und/oder verklebt.

Die vordere Stützstruktur stellt vorteilhaft eine Haltefunktion der Filtermembran bereit.

Die hintere und/oder vordere Stützstruktur(en) können derart ausgestaltet sein, dass nach Anordnen der Stützstruktur(en) noch unabgestützt frei zugängliche Flächenanteile der Filtermembran eine jeweils hinreichend kleine Ausdehnung bis zu den benachbarten mechanischen Abstützungen oder Stützstrukturen aufweisen.

Der maximal zulässige Fluiddruck des zweiten Fluids auf die freien Filtermembranflächen erzeugt damit bevorzugt keine unzulässig hohe Spannung (beispielsweise bedingt durch Ausbeulung der Filtermembran) mehr.

Beispielsweise können einzelne oder alle Stützstrukturen, wie beispielsweise Drainagestrukturen, eine Breite von etwa 0,5 bis 2 mm aufweisen.

Der außenseitig angeordnete Fluidkonnektor kann über Bohrungen, Aussparungen oder Öffnungen mit der außenseitigen Drainage- oder Stützstruktur, d. h. der hinteren Stützstruktur, für die Filtermembran in Verbindung stehen.

Die zwischen der hinteren und der vorderen Stützstruktur angeordnete Filtermembran kann im Wesentlichen bis auf ein gebrauchsbestimmt erforderliches Maß mechanisch belastbar sein.

Die Ebene, in welcher eine derartige Membranverbindung, d. h. eine Verbindung zwischen der Filtermembran und den beiden Stützstrukturen, angeordnet ist, kann im Wesentlichen der Ebene entsprechen, in welcher erhabene Bereiche der Drainagestruktur angeordnet sind.

Je nach Verbindungsverfahren und/oder Dicke der Filtermembran kann ein Höhenversatz zwischen erhabenen Bereichen der Drainagestruktur und der außenseitigen Ebene der Filtermembran sinnvoll sein. Ein solcher Höhenversatz kann dazu dienen, die außenseitige Ebene der Filtermembran kraftfrei und/oder mit leichtem Spiel auf den erhabenen Drainagestrukturen aufliegen zu lassen.

Die erhabenen Drainagestrukturen können so klein wie möglich angeordnet sein.

Das zweite Fluid kann vorzugsweise nur beschränkt durch die Filtermembranbereiche strömen, welche durch Anlage an die erhabenen Drainagestrukturen in Kontakt und/oder in Verpressung mit diesen gelangen.

Die Größe und/oder Anzahl der Verbindungsbohrungen zum Fluidkonnektor und/oder die Anordnung, Anzahl, Breite und/oder Tiefe der vertieften Drainagestrukturen können derart sein, dass ein möglicher durch diese Strömungswege verursachter Druckabfall des zweiten Fluids ein vernachlässigbarer oder hinnehmbarer Bruchteil des Gesamtdruckabfalls beim Durchströmen der Filtereinrichtung ist.

Die Breite der vertieften Drainagestrukturen und/oder die Durchmesser der Verbindungsbohrungen zum Fluidkonnektor können hinreichend klein ausgelegt sein derart, dass die bei maximal möglichen Druckdifferenzen auf die Filtermembran wirkenden Zugkräfte (welche beispielsweise zu einer Ausbeulung in die vertieften Strukturen führen) deutlich unterhalb der zulässigen Zugkräfte bevorzugt sowohl innerhalb der Filtermembran als auch an den in der Regel empfindlicheren Zonen am Übergang zur Ringbefestigung (zum Beispiel Schweißnaht) liegen.

In einigen, beispielhaften erfindungsgemäßen Ausführungsformen weisen die hintere und die vordere Stützstruktur zu einer Hauptebene der Membran spiegelbildlich identische oder im Wesentlichen identische Drainagestrukturen auf.

Bevorzugt liegen sich die vertieften und/oder die erhabenen Drainagestrukturen deckungsgleich oder im Wesentlichen deckungsgleich gegenüber.

Bevorzugt sind die vertieften Drainagestrukturen auf der einen Filtermembranseite schmäler bzw. die erhabenen Drainagestrukturen auf dieser Filtermembranseite breiter ausgeführt als die Drainagestrukturen auf der anderen Filtermembranseite. Dies kann eine größere laterale Einbautoleranz erlauben. Die Teilungsabstände und/oder Anordnungen der Strukturen können jedoch auf beiden Seiten bevorzugt identisch ausgeführt sein.

Auf diese Weise kann sich bei Ausnutzung der lateralen Einbautoleranzen ein sehr konstantes Eigenschaftsprofil hinsichtlich Merkmalen wie Fluiddurchtrittswiderstand und Grad der mechanischen Abstützung ergeben.

Die Gesamtstärke der Struktur der vorderen Stützstruktur kann sich aus der Tiefe der Drainagestruktur und/oder der minimal möglichen Wandstärke des Materials der vorderen Stützstruktur ergeben oder die Summe hieraus sein. Dadurch kann vorteilhafter Weise die vordere Stützstruktur wenig Bauraum benötigen und/oder günstiger hergestellt werden.

Ein weiterer Vorteil kann darin bestehen, dass an die vordere Stützstruktur keine besonderen Anforderungen an Präzision und Steifigkeit bestehen. Zudem kann es vorteilhaft möglich sein, die vordere Stützstruktur an der Fluidzuführungskammer einzig nach Kostengesichtspunkten und/oder möglichst geringem Aufwand zu befestigen.

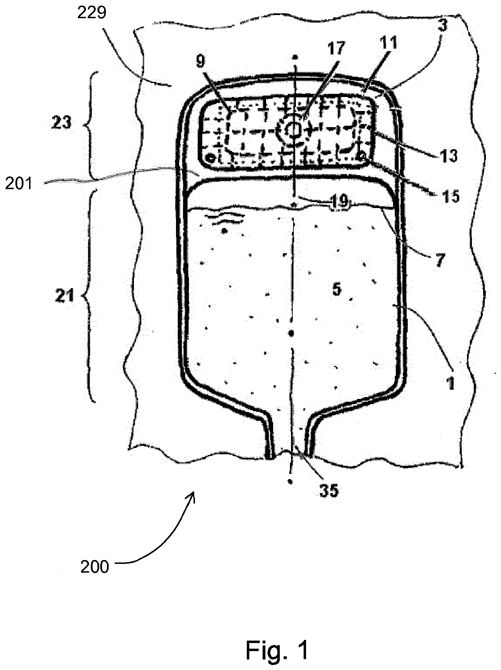

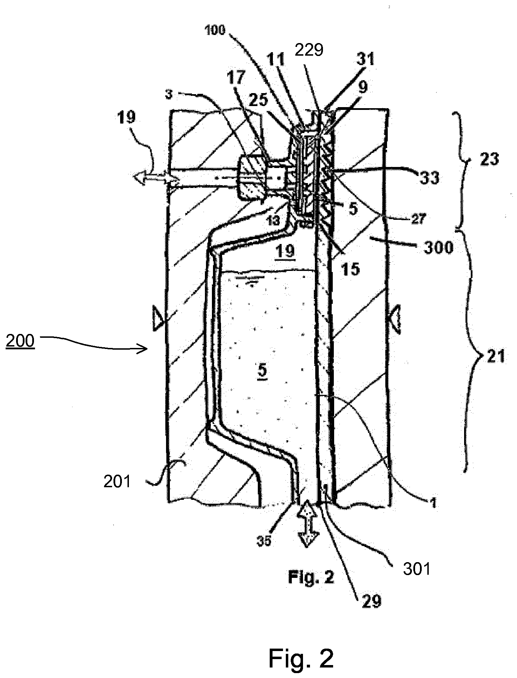

Wie in den Fig. 1 und 2 gezeigt ist, kann die vordere Stützstruktur beispielsweise mittels einer Steck- oder Nietbefestigung oder dem Prinzip der Bolzenhalterung mit der Fluidzuführungskammer verbunden sein. Ebenso kann die vordere Stützstruktur mit der Fluidzuführungskammer mittels punktförmigem Anschweißen und/oder Einklipsen in geeignete geometrische Ausgestaltungen einer Seitenwand oder Wandung der Fluidzuführungskammer verbunden sein.

Die Drainagestrukturen der vorderen Stützstruktur können sich in einigen, beispielhaften erfindungsgemäßen Ausführungsformen von den außenseitigen Drainagestrukturen u.a. oder allein darin unterscheiden, dass erstere nach außen hin nicht an den Membrangrenzen enden, sondern radial nach außen bis an die Bauteilberandung durchgeführt sind. Damit können ein- und/oder ausströmende Fluide ungehindert in den verbleibenden Ringraum zwischen der vorderen Stützstruktur und einer oberen Berandung bzw. einem oberen Rand der Fluidzuführungskammer dringen. Die Fluide können vorteilhaft großlumig mit der Fluidaufnahmekammer in Verbindung stehen.

In einigen, beispielhaften erfindungsgemäßen Ausführungsformen weist die Fluidaufnahmekammer eine erste Bauhöhe auf, und die Fluidzuführungskammer weist eine zweite Bauhöhe auf, welche von der ersten Bauhöhe verschieden ist.

Auch oder zusätzlich dazu kann die Fluidzuführungskammer während der Verwendung der medizinischen Einrichtung oberhalb der Fluidaufnahmekammer ("oben") angeordnet sein. "Oberhalb" kann sich auf ein als durch den Erdmittelpunkt verlaufend gedachtes Bezugssystem beziehen.

In einer derartigen Anordnung der Fluidaufnahmekammer und der Fluidzuführungskammer ist die medizinische Einrichtung bevorzugt in gestufter Tiefe ausgeführt. Die während der Verwendung der medizinischen Einrichtung unterhalb der Fluidzuführungskammer ("unten") angeordnete tiefe Fluidaufnahmekammer kann im Gebrauch als Reservoir und/oder Behandlungsraum für die sich darin befindenden Fluide genutzt werden.

Die erfindungsgemäße medizinische Einrichtung kann an wenigstens einer Seite mit einem Abdeckungselement versehen sein.

Besonders bevorzugt sind die Filtereinrichtung und/oder die Filtermembran parallel oder im Wesentlichen parallel zum Abdeckungselement der erfindungsgemäßen medizinischen Einrichtung angeordnet.

In einigen beispielhaften erfindungsgemäßen Ausführungsformen ist das zweite Fluid ein Gas. Weiter bevorzugt kann das erste Fluid eine Flüssigkeit, wie beispielsweise Blut, sein.

Die erfindungsgemäße medizinische Einrichtung ist zum Einsatz in bzw. an bzw. mit einer Behandlungsvorrichtung, wie einer medizinischen Behandlungsvorrichtung, einer Vorrichtung aus der Labortechnik, einer Vorrichtung der Nahrungsmittel- und/oder Arzneimittelherstellung geeignet. Zum Einbringen bzw. Einleiten oder Einführen in die erfindungsgemäße Aufnahmeeinrichtung geeignete Fluide können daher sowohl medizinische Flüssigkeiten wie Blut, Substituat (z. B. Kochsalzlösung), Wirkstoffzubereitungen, wie Lösungen, Suspensionen, Emulsionen, Trägergase für Wirkstoffe, Reinigungsflüssigkeiten oder -gase, Desinfektionsflüssigkeiten oder -gase, Sterilisationsflüssigkeiten oder -gase, Getränkeflüssigkeiten und dergleichen einschließen.

Wenn die Filtermembran als Sterilmembran vorgesehen ist, kann die erfindungsgemäße medizinische Einrichtung insbesondere zur Sterilluftversorgung der Fluidaufnahmekammer eingesetzt werden.

Eine erfindungsgemäße medizinische Einrichtung kann eine Einmalkomponente oder ein Einmalartikel sein, welcher beispielsweise aus einem Kunststoffmaterial gefertigt ist.

Die erfindungsgemäße medizinische Einrichtung kann mittels eines Spritzgussverfahrens hergestellt sein.

Die erfindungsgemäße medizinische Einrichtung kann über Flüssigkeits- und/oder Gasanschlüsse, über halboffene Kanäle und/oder Kammern und/oder über Strukturen zur Ankopplung an Aktoren und/oder Sensoren verfügen. Derartige Aktoren und/oder Sensoren können zur Durchführung von bevorzugt nicht-invasiven und/oder steril entkoppelten Funktionen an den Flüssigkeiten in der Kassette dienen. Eine oder mehrere Abdeckungselemente, wie beispielsweise Membranen, insbesondere preisgünstige Folien, können für den Abschluss und/oder die Abdichtung der Kanäle und Kammern sorgen.

In bestimmten, beispielhaften erfindungsgemäßen Ausführungsformen sind die Filtereinrichtung oder die Filtermembran der medizinischen Einrichtung parallel zu einem Abdeckungselement der medizinischen Einrichtung zum Verschließen eines Inneren der Fluidaufnahmekammer gegenüber einem Äußeren angeordnet.

Beispielsweise kann die Blutbehandlung, für welche die medizinische Einrichtung verwendet wird, ein Dialysierverfahren, eine Hämodialyse, Hämofiltration, Hämodiafiltration und dergleichen, sein.

Die erfindungsgemäße medizinische Einrichtung kann in vorteilhafter Weise zur Sterilluftversorgung einer Fluidaufnahmekammer eingesetzt werden.

In bestimmten, beispielhaften erfindungsgemäßen Ausführungsformen haben die Fluidzuführungskammer und die Fluidaufnahmekammer zusammen nur genau zwei Verbindungen zu einem Äußeren außerhalb dieser beiden Kammern.

In bestimmten, beispielhaften erfindungsgemäßen Ausführungsformen ist die Filtermembran nicht in einem Schlauchabschnitt angeordnet.

Ein Schlauch kann eine über seinen gesamten Querschnitt flexible Leitung für ein Fluid sein.

In bestimmten, beispielhaften erfindungsgemäßen Ausführungsformen besteht neben der Fluidzuführungskammer und der Fluidaufnahmekammer keine Schlauchverbindung.

In bestimmten, beispielhaften erfindungsgemäßen Ausführungsformen sind weder die Fluidzuführungskammer noch die Fluidaufnahmekammer flexible oder vollkommen flexible Beutel.

In bestimmten, beispielhaften erfindungsgemäßen Ausführungsformen ist die medizinische Einrichtung keine Vorrichtung zum selektiven Blockieren der Passage von Fetten.

In bestimmten, beispielhaften erfindungsgemäßen Ausführungsformen weisen die medizinische Einrichtung, die Fluidzuführungskammer und/oder die Fluidaufnahmekammer keinen Einsteckdorn auf.

In bestimmten, beispielhaften erfindungsgemäßen Ausführungsformen liegt der Zugang für das zweite Fluid zur Fluidzuführungskammer auf einer Seite der Filtermembran (bezogen auf einen Strömungsweg durch die Filtermembran) und der Zugang für das erste Fluid zur Fluidaufnahmekammer auf der anderen Seite hiervon.

In bestimmten, beispielhaften erfindungsgemäßen Ausführungsformen ist die Fluidaufnahmekammer mit einem Fluidkanal der medizinischen Einrichtung unmittelbar verbunden, wobei der Fluidkanal zumindest abschnittsweise durch den Einrichtungskörper verläuft oder ausgebildet wird.

In bestimmten, beispielhaften erfindungsgemäßen Ausführungsformen ist die Fluidaufnahmekammer strömungstechnisch durch die Filtermembran begrenzt.

In bestimmten, beispielhaften erfindungsgemäßen Ausführungsformen liegen die Fluidzuführungskammer und die Fluidaufnahmekammer im Einrichtungskörper der medizinischen Einrichtung vor.

In bestimmten, beispielhaften erfindungsgemäßen Ausführungsformen ist die medizinische Einrichtung eine Blutkassette. Der Einrichtungskörper ist in diesen Fällen ein Kassettenkörper.

In bestimmten, beispielhaften erfindungsgemäßen Ausführungsformen weist die medizinische Einrichtung ferner wenigstens zwei Konnektoren für Pumpschlauchsegmente für peristaltische Pumpen, mit oder ohne die Pumpschlauchsegmente auf.

In bestimmten, beispielhaften erfindungsgemäßen Ausführungsformen weist die medizinische Einrichtung zudem einen arteriellen Patientenanschluss, eine arterielle Filterleitung, einen venösen Patientenanschluss und eine arterielle Heparin-Zugabestelle auf. Die arterielle Heparin-Zugabestelle ist zwischen der arteriellen Filterleitung und dem venösen Patientenanschluss angeordnet.

In bestimmten, beispielhaften erfindungsgemäßen Ausführungsformen weist die medizinische Einrichtung eine venöse Filterleitung und eine venöse Heparin-Zugabestelle auf, wobei die venöse Filterleitung medial zur venösen Heparin-Zugabestelle angeordnet ist.

In bestimmten, beispielhaften erfindungsgemäßen Ausführungsformen weist die medizinische Einrichtung eine Single-Needle-Sterilmembran auf, welche die Form eines Parallelogramms hat.

Die Verwendung als Single-Needle- Sterilmembran stellt eine besondere Anforderung an die Filtermembran dar, da dort wiederholt ein Druck von der Rückseite der Filtermembran aufgebaut wird, um das Blut des Fluidsystems dem Patienten zuzuführen. In dieser Funktion kann die Filtermembran sowohl einen Schutz für die Maschine durch ein Verhindern eines Eintretens von Flüssigkeit in die Maschine als auch einen Schutz für den Patienten durch Verhindern von Verschmutzungen des Fluidsystems bewirken.

In einer bevorzugten Ausführungsform weist die medizinische Einrichtung ein primäres oder erstes Ausrichtungszentrum und ein sekundäres oder zweites Ausrichtungszentrum auf, etwa in Form von Zentrieröffnungen. Das primäre Ausrichtungszentrum und das sekundäre Ausrichtungszentrum weisen unterschiedliche Formen und/oder unterschiedliche Ausrichtungen auf.

Einige oder alle erfindungsgemäßen Ausführungsformen können einen oder mehrere der oben oder im Folgenden genannten Vorteile aufweisen.

Die erfindungsgemäße medizinische Einrichtung kann aufgrund seiner hierin beschriebenen vorderen Stützstruktur in einigen erfindungsgemäßen Ausführungsformen mittels Strahlensterilisation oder "e-beam" sterilisiert werden. Strahlensterilisation und "e-beam" können zu einer Delamination der zumeist aus dünner Teflonschicht und dickerem Trägervlies gefertigten Filtermembran führen. Dadurch wird die Festigkeit der Filtermembran gegen Druck gesenkt. Übersteigt der Druck einen vergleichsweise geringen Wert, so kann sich die Teflonschicht vom Trägervlies lösen (oder umgekehrt), was sowohl für die Sicherheit des Patienten als auch für die Funktion der Filtermembran oder der medizinischen Einrichtung abträglich sein kann und hierin als Delamination bezeichnet wird. Die Delamination tritt bevorzugt am Schweißabschnitt, an welchem die Filtermembran mit dem Einrichtungskörper verschweißt ist, auf. Die vordere Stützstruktur steht erfindungsgemäß mit dem Schweißabschnitt in Kontakt. Es ist dieser Kontakt, der einer Delamination bei gegebenem Druck entgegenwirken kann oder eine Druckbelastung im Gebrauch mit einem um den Faktor 4 höheren Druck ohne Auftreten von Delamination zulässt. Von Bedeutung ist dabei, dass die Stützung der Filtermembran an deren Vorderseite erfolgt.

Von Vorteil ist ferner, wenn der Kontakt zwischen dem ersten Auflageabschnitt und der Filtermembran sich über den Schweißabschnitt hinaus zu einem Inneren der Filtermembran erstreckt. Auch dies kann vorteilhaft einer Delamination entgegenwirken.

Anders als bei herkömmlichen Anordnungen zur Sterilluftversorgung von externen Funktionseinrichtungen, bei welchen die hydrophoben Sterilmembranen am höchsten Punkt der Fluidaufnahmekammer und im Wesentlichen parallel zur freien Fluidoberfläche angeordnet sind, kann die erfindungsgemäß vorgesehene Filtermembran bevorzugt geodätisch oberhalb der normal maximal mit Fluiden gefüllten Fluidaufnahmekammer angeordnet sein. Im störungsfreien Betrieb kann die Fluidzuführungskammer daher vorteilhaft nur mit dem zweiten Fluid, zum Beispiel Gas, und mit geringen Mengen des ersten Fluids, z.B. einer Flüssigkeit wie Blut, in Kontakt kommen.

Die erfindungsgemäß eingesetzten Filtermembranen können als Single-Needle-Filtermembran ausgestaltet sein.

Die erfindungsgemäß eingesetzten Filtermembranen können die Form eines Parallelogramms haben.

Da die erfindungsgemäß eingesetzten Filtermembranen als empfindliche und schwer überprüfbare Elemente in der Regel mit jedem Behandlungseinsatz ausgewechselt werden sollten, kann die Filtermembran vorteilhaft Bestandteil der medizinischen Einmal-Einrichtung sein. Derartige Filtermembranen können ferner weiter vorteilhaft zugleich gemeinsam mit der medizinischen Einmal-Einrichtung sterilisiert werden und/oder das Einmalteilsystem bei Lagerung und/oder während der Konnektion mit der Behandlungsvorrichtung steril verschlossen halten.

Hydrophobe Filtermembranen können ferner vorteilhaft dafür sorgen, dass bei Fehlsteuerungen die in der Fluidaufnahmekammer vorhandenen, zu behandelnden Fluide nicht in die Behandlungsvorrichtung gelangen können.

Die erfindungsgemäße medizinische Einrichtung kann vorteilhaft die in für Behandlungsvorrichtungen, insbesondere Blutbehandlungsvorrichtungen, oftmals vorgesehenen medizinischen Einmal- Einrichtungen vorhandenen räumlichen und/oder funktionalen Anordnungen zur Unterbringung, zur Verpressung, zum Toleranzausgleich, zur Kraftbegrenzung, zur Halterung, zur Ausrichtung und/oder zur Handhabung parallel zu anderen Funktionseinheiten der Gesamtanordnung nutzen.

Aus den unterschiedlichen geometrischen Umgebungsbedingungen herkömmlicher Anordnungen kann mit der Anordnung der Fluidzuführungskammer und der Fluidaufnahmekammer der erfindungsgemäßen medizinischen Einrichtung durch räumliche Enge im Umfeld der Filtermembran vorteilhaft ein flächenspezifisch höherer Gasdruckverlust an den Drainage- oder Stützstrukturen ergeben. Auf diese Weise kann es vorteilhaft möglich sein, bei gleichem Druckverlust, preisgünstigere und kleiner gebaute Filtermembranen einzusetzen, was aufgrund der Rechteckform häufig zugleich den Bauraum effektiver zu nutzen vermag.

Während aus Festigkeitsgründen der umgebenden Stützkonstruktion in herkömmlichen Systemen die Filtermembran in der Regel rund ausgeführt werden muss und mit geringer lateraler Toleranz in die Gehäuseumgebung eingepasst werden muss, kann die Filtermembran in der erfindungsgemäßen medizinischen Einrichtung verschnittfrei als Rechteck aus einem Filtermembranband hergestellt werden und unter großzügiger lateraler Toleranz auf die Wandung der Fluidzuführungskammer aufgeschweißt, aufgeklebt oder aufgepresst werden.

Im Gegensatz zu Schweißverbindungen zwischen gleichartigen oder beidseitig aufschmelzenden Verbindungspartnern, bei denen sich zwar der gleiche Dichteffekt zwischen den Stützstrukturen und der Filtermembran ergeben würde, aber mit einer geringeren mechanischen Belastbarkeit der Verbindung einhergehen, kann erfindungsgemäß durch Auswählen eines niederschmelzenden Materials für die Fluidzuführungskammer eine dichte und zugleich mechanisch gut belastbare Verbindung erreicht werden. Auf diese Weise kann vorteilhaft vermieden werden, dass die erste Stützschicht durch das Aufschmelzen und Verpressen dünner wird. Ferner können vorteilhaft Materialfehler und/oder ungünstige Querschnittssprünge an den Übergängen zu den nicht aufgeschmolzenen Bereichen der Filtermembran vermieden werden.

Weiterhin ist mit der erfindungsgemäß vorgesehenen vorderen Stützstruktur eine gute Zugänglichkeit der Filtermembran aus seitlicher Richtung, in der Ebene der Filtermembran, möglich. Dies kann insbesondere bei ungewollter Benetzung der Filtermembran mit Flüssigkeit von Vorteil sein.

Durch die höhere Drainagefähigkeit zum Inneren der Fluidaufnahmekammer hin können sich vorteilhaft wichtige Sicherheitsvorteile ergeben: Bei herkömmlichen Filtermembrananordnungen mit zur Flüssigkeitsoberfläche parallelen Filtermembranen kann es bei unzulässiger vollständiger Flutung bis an die Filtermembran zu einem Druckstoß kommen, welcher sowohl zur Filtermembranzerstörung wie auch zur Zerstörung oder Irritation weiterer Komponenten der medizinischen Einrichtung und/oder der Behandlungsvorrichtung führen kann. Mit der erfindungsgemäßen medizinischen Einrichtung kann jedoch ein unzulässig weiter steigender Pegel kontinuierlich die Filtermembranfläche überstreichen, so dass sich der Durchflussdruck sanft ansteigend und stoßfrei erhöhen kann.

Daneben kann die erfindungsgemäß vorgesehene Anordnung der Filtereinrichtung vorteilhaft einer mehrfachen gefahrlosen Wiederholbarkeit des Fehlerfalls einer vollständigen Flutung der Fluidaufnahmekammer Stand halten, da die Flüssigkeit in der vorliegenden Erfindung in der Lage ist, unter Schwerkrafteinfluss selbsttätig wieder von der Filtermembran in die Fluidaufnahmekammer abzulaufen.

Auf diese Weise kann es vorteilhaft möglich sein, einen Abbruch des Behandlungsverfahrens zu vermeiden.

Im Folgenden wird die vorliegende Erfindung unter Bezugnahme auf die beigefügte Zeichnung beschrieben. In der Zeichnung bezeichnen identische Bezugszeichen gleiche oder identische Elemente. Es zeigen:
- Fig. 1: eine Draufsicht auf einen Ausschnitt der medizinischen Einrichtung, hier exemplarisch als Blutkassette ausgestaltet;
- Fig. 2: einen Querschnitt durch eine Blutkassette;
- Fig. 3: einen Ausschnitt der Blutkassette aus Fig. 2 in Vergrößerung im Längsschnitt, gekippt um 90° gegenüber der Fig. 2 und gegenüber einer Anordnung beim Gebrauch der Blutkassette;
- Fig. 4a: eine vordere Stützstruktur mit erstem ersten Auflageabschnitt;
- Fig. 4b: Ausschnitte der vorderen Stützstruktur der Fig. 4a in Vergrößerung;
- Fig. 5a-5d: schematisch sehr vereinfacht und nur in Ausschnitten die Blutkassette in verschiedenen, beispielhaften Ausführungsformen;
- Fig. 6a-6d: Abschnitte sowohl des Kassettenkörpers als auch der vorderen Stützstruktur in unterschiedlichen, beispielhaften Ausführungsformen der Blutkassette;
- Fig. 7a-7d: verschiedene Ausführungsformen des zweiten Auflageabschnitts und des Stützabschnitts der Blutkassette;
- Fig. 8: eine Einrichtung mit einer zweiten Filtermembran, die anstelle der vorderen Stützstruktur vorgesehen ist;
- Fig. 9: wie Fig. 8 eine Ausführungsform mit einer zweiten Filtermembran;
- Fig. 10: eine weitere Ausführungsform der vorderen Stützstruktur vor einem Aufschmelzen von Abschnitten hiervon;
- Fig. 11: eine weitere Ausführungsform der vorderen Stützstruktur nach einem Aufschmelzen von Abschnitten hiervon; und
- Fig. 11a: einen vergrößerten Teilausschnitt der Fig. 11.

**Fig. 1** zeigt eine Draufsicht auf einen Ausschnitt einer medizinischen Einrichtung, welche exemplarisch als Blutkassette 200 ausgestaltet ist, gemäß einer exemplarischen Ausführungsform der vorliegenden Erfindung.

Obgleich die medizinische Einrichtung hier exemplarisch als Blutkassette 200 ausgestaltet ist und mit Bezug auf diese diskutiert wird, soll die vorliegende Erfindung nicht hierauf beschränkt werden. Die medizinische Einrichtung kann beispielsweise ein Schlauchsystem, ein Schlauchset oder jeweils ein Teil hiervon sein.

Die Blutkassette 200 weist einen Kassettenkörper 201 auf. Die Blutkassette 200 weist ferner optional ein Abdeckungselement 229, hier in Gestalt einer Folie, auf. Das Abdeckungselement 229, soweit vorhanden, bedeckt den Kassettenkörper 201 zumindest abschnittsweise, möglicherweise an einer gesamten Seite hiervon. Das Abdeckungselement 229 ist aus weicherem Material gefertigt als der Kassettenkörper 201, weshalb letzterer hierin auch als Hartteil bezeichnet wird. Das Abdeckungselement 229 deckt den Kassettenkörper 201 gegenüber einem Äußeren oder einer Umgebung ab.

Der Kassettenkörper 201 weist eine Fluidaufnahmekammer 1 sowie optional eine Fluidzuführungskammer 3 auf.

In der Fluidaufnahmekammer 1 befindet sich im Gebrauch zeitweise ein erstes medizinisches Fluid 5, welches die Fluidaufnahmekammer 1 in Fig. 1 bis zu einem Fluidspiegel oder -pegel 7 füllt.

Es ist optional die Fluidzuführungskammer 3, welche eine Filtereinrichtung aufweist, von welcher in Fig. 1 eine Filtermembran 9 sowie eine wiederum optional vorgesehene, hintere Stützstruktur 11 gezeigt sind.

Die hintere Stützstruktur 11 weist eine Drainagestruktur 13 auf. Die hintere Stützstruktur 11 ist über Befestigungseinrichtungen 15, beispielsweise Nietverbindungen, Schweißnähte oder dergleichen, mit dem Kassettenkörper 201, etwa mit der Fluidzuführungskammer 3, verbunden.

Ein Fluidkonnektor 17 ist mit der hinteren Stützstruktur 11 der Filtereinrichtung verbunden.

Durch den Fluidkonnektor 17 wird ein zweites Fluid 19 in die Fluidaufnahmekammer 1 eingebracht. Das zweite Fluid kann zum Behandeln des ersten Fluids 5, beispielsweise durch Aufbringen von Druck, verwendet werden.

Wie in Fig. 1 gezeigt, stellt die Fluidaufnahmekammer 1 einen unteren Bereich 21 und die Fluidzuführungskammer 3 einen oberen Bereich 23 dar.

Der in Fig. 1 gezeigte Ausschnitt der Blutkassette 200 ist in Fig. 1 derart angeordnet, dass der Blick auf die Vorderseite der Filtermembran 9 fällt. Die Filtermembran 9 liegt mit ihrer hinter der Zeichenebene liegenden Rückseite auf der hinteren Stützstruktur 11. Letzte wird als "hintere" Stützstruktur bezeichnet, da sie der Rückseite der Filtermembran 9 zugewandt ist oder auf der Rückseite der Filtermembran 9 liegt.

**Fig. 2** zeigt eine Blutkassette 200 im Querschnitt. Die Blutkassette 200 ist mit einer Behandlungsvorrichtung 300 verpresst.

Der untere Bereich 21 bildet den tieferen Bereich, also die Fluidaufnahmekammer 1. Der obere Bereich 23 bildet den flacheren Bereich, also die Fluidzuführungskammer 3.

Wie in Fig. 2 mit dem Doppelpfeil gezeigt ist, wird ein zweites Fluid 19 durch einen Fluidkonnektor 17 in die Fluidzuführungskammer 3 eingebracht.

Die Fluidzuführungskammer 3 weist, wie in Fig. 2 gezeigt ist, optional die hintere Stützstruktur 11 auf, welche mittels einer dichten Verbindung 25, beispielsweise einer Schweiß- oder Klebverbindung, an der Fluidzuführungskammer 3 befestigt ist.

Auf der hinteren Stützstruktur 11 ist die Filtermembran 9 angeordnet, auf welcher wiederum eine vordere Stützstruktur 27 vorgesehen ist.

Der Kassettenkörper 201 der Blutkassette 200 ist an einer Seite mit einem Abdeckungselement 229, beispielsweise einer Folie, abgedeckt.

Über eine Gummimatte 301, welche zum Übertragen von Kraft und/oder Bewegungen durch Sensoren oder Aktoren der Behandlungsvorrichtung 300 auf die Blutkassette 200 bzw. Kammern und/oder Kanäle derselben dienen kann, wird die Blutkassette 200 mit der Behandlungsvorrichtung 300 verpresst.

Die Gummimatte 301 kann eine gezielte Nachgiebigkeitsstruktur 33 aufweisen.

Zum Einbringen oder Entfernen der sich im Inneren der Fluidaufnahmekammer 1 befindenden Fluide kann die Fluidaufnahmekammer 1 an ihrer Unterseite mit einem Fluidanschluss 35 versehen sein.

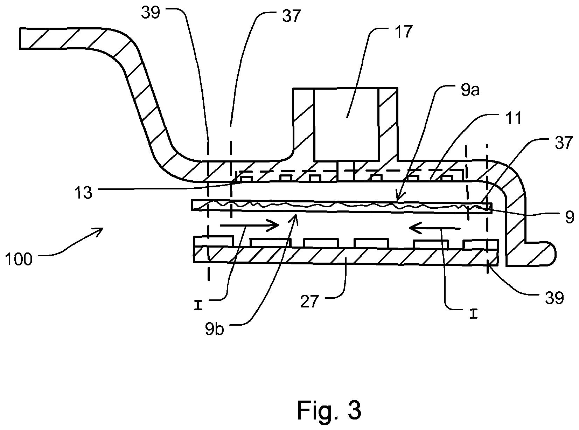

**Fig. 3** zeigt einen vergrößerten Ausschnitt bzw. Abschnitt der Blutkassette 200 aus Fig. 2, genauer gesagt, die Filtereinrichtung 100 im Längsschnitt.

Im Vergleich zur Darstellung der Fig. 2 wurde die Filtereinrichtung 100 um 90° gekippt, so dass der Fluidkonnektor 17 für das zweite Fluid in der Darstellung der Fig. 3 nach oben gerichtet ist.

Die Kippung dient der besseren Darstellung. Im Gebrauch der Blutkassette 200 nimmt die Filtereinrichtung 100 ihre in Fig. 1 gezeigte Lage ein.

Die Filtermembran 9, welche exemplarisch eine Membranschicht und eine (nicht dargestellte) dritte Stützschicht in Form eines Gewebes aufweist, ist zwischen der hinteren Stützstruktur 11 und der vorderen Stützstruktur 27 angeordnet.

Zur detaillierten Beschreibung der einzelnen Komponenten wird auf die obigen Ausführungen verwiesen.

Zum Herstellen der dichten Verbindung zwischen der hinteren Stützstruktur 11 und der Filtermembran 9, ist eine optionale erste Ringzone bzw. ein erster Ringbereich 37 vorgesehen. Zum Herstellen der Verbindung zwischen der Filtermembran 9 und der vorderen Stützstruktur 27 ist eine optionale zweite Ringzone bzw. ein zweiter Ringbereich 39 vorgesehen.

Die Pfeile I der Fig. 3 zeigen zum inneren oder zentralen Abschnitt der Filtermembran 9. Sie zeigen vom Rand der Filtermembran 9 nach innen.

Das Bezugszeichen 9a bezeichnet die Rückseite der Filtermembran 9, das Bezugszeichen 9b die Vorderseite der Filtermembran 9.

Die Filtermembran 9 kann ein Trägervlies auf ihrer Rückseite 9a und/oder ihrer Vorderseite 9b tragen. Ein Trägervlies kann ein Gewebe sein, es kann eine Teflonschicht (alternativ: eine Polytetrafluorethylen-(PTFE)-schicht) aufweisen.

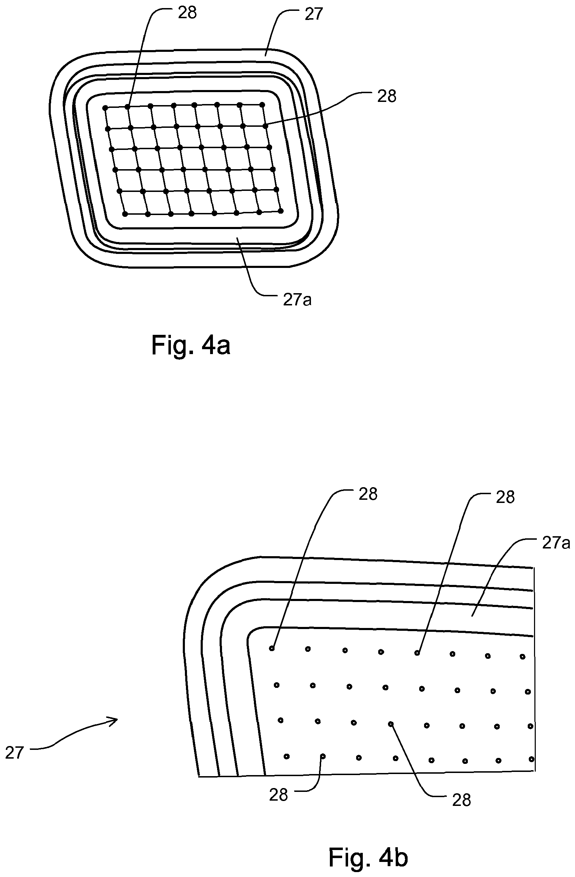

Fig. 4a zeigt die vordere Stützstruktur 27 mit einem ersten Auflageabschnitt 27a. Die Darstellung der Fig. 4a entspricht dem Blick auf jene Seite der vorderen Stützstruktur 27, welche im Gebrauch wiederum der Vorderseite 9b der Filtermembran 9 zugewandt ist. Die vordere Stützstruktur 27 weist durch sie hindurchführende Öffnungen 28 auf, welche nachfolgend mit Bezug auf Fig. 4b erläutert sind.

Der erste Auflageabschnitt 27a kann, wie in Fig. 4a exemplarisch gezeigt, aus einem anderen Material als die übrigen Abschnitte oder andere Abschnitte der vorderen Stützstruktur 27 gefertigt sein. Beispielsweise kann der erste Auflageabschnitt 27a aus Silikon sein oder Silikon aufweisen und optional, etwa in Form eines O-Rings, in eine Nut oder in einen Schlitz der vorderen Stützstruktur 27 eingesetzt werden. Die vordere Stützstruktur 27 kann somit im Wesentlichen aus einem vergleichsweise günstigen und härteren Material gefertigt sein. Zugleich kann ihr erster Auflageabschnitt 27a aus einem weicheren Material gefertigt sein kann, das zwar verglichen mit dem härteren Material teurer ist, bei seiner Auflage auf die Filtermembran 9 und bei Verpressen der vorderen Stützstruktur 27 gegen die Filtermembran 9 anders als das härtere Material aber vorteilhafter Weise zu keinen Schäden führen kann.

Der erste Auflageabschnitt 27a kann alternativ - anders als in Fig. 4a gezeigt - erfindungsgemäß aus demselben Material wie die übrigen Abschnitte oder andere Abschnitte der vorderen Stützstruktur 27 sein. Beispielsweise kann die gesamte vordere Stützstruktur 27 einschließlich des ersten Auflageabschnitts 27a aus Silikon sein oder aus Silikon bestehen. Dies bringt fertigungstechnische Vorteile bei allerdings höheren Materialkosten, da Silikon kein kostengünstiger Werkstoff ist.

Von der vorliegenden Erfindung umfasst sind ferner Ausführungsformen, bei welchen - wiederum anders als in Fig. 4a gezeigt - alle Abschnitte der vorderen Stützstruktur 27 aus einem Material gefertigt sind oder hieraus bestehen, wobei es sich bei diesem Material nicht um Silikon handelt. Auf dieses Weise kann die vordere Stützstruktur 27 kostengünstig gefertigt werden. Die gewünscht niedrige Härte der vorderen Stützstruktur 27 wird beim Verbinden der vorderen Stützstruktur 27 mit dem Kassettenkörper 201 beim Verschweißen und durch das Verschweißen der vorderen Stützstruktur 27 im Bereich des ersten Auflageabschnitts 27a mit dem Kassettenkörper 201 erreicht.

**Fig. 4b** zeigt einen vergrößerten Ausschnitt der vorderen Stützstruktur 27 der Fig. 4a.

Zu erkennen sind Öffnungen 28, welche die vordere Stützstruktur 27 durchlässig machen für das Fluid, welche auch die Filtermembran 9 durchtreten darf. Die Durchlässigkeit der vorderen Stützstruktur 27 für das Fluid kann optional allein durch die Öffnungen 28 bedingt sein wie im vorliegenden Beispiel.

Vorzugsweise beträgt der Durchmesser (aller oder vieler) der Öffnungen 28 maximal 0,7 mm, vorzugsweise nicht mehr als 0,5 mm. Sind die Öffnungen 28 als Langlöcher ausgestaltet, so gelten die vorstehenden Maße analog für die Breite der Langlöcher.

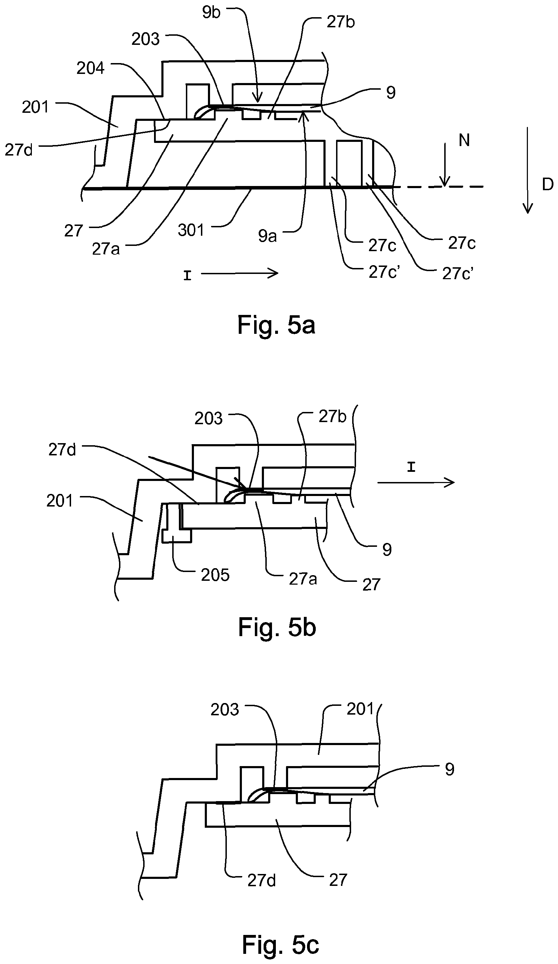

**Fig. 5a** zeigt schematisch sehr vereinfacht und nur in Ausschnitten die Blutkassette 200 einer beispielhaften erfindungsgemäßen Ausführungsform mit dem Kassettenkörper 201 (ist wie in den Fig. 5b bis 5d um 180° seitenverdreht gezeigt gegenüber seiner Darstellung in Fig. 3), einer Filtermembran 9, einer vorderen Stützstruktur 27 und einer Gummimatte 301. Die Gummimatte 301 ist Teil einer in Fig. 5a nicht gezeigten Blutbehandlungsvorrichtung 300, in welche die Blutkassette 200 aufgenommen ist.

Die Filtermembran 9 ist mit dem Kassettenkörper 201 entlang eines Schweißabschnitts 203 verschweißt. Der Schweißabschnitt 203 kann umlaufend geschlossen verlaufen, was aufgrund der Schnittdarstellung in Fig. 5a dieser Figur nicht zu entnehmen ist. Der Schweißabschnitt 203 kann als erster Ringbereich 37 verstanden werden, siehe Fig. 2.

Die vordere Stützstruktur 27 liegt mit einem ersten Auflageabschnitt 27a zumindest teilweise oder abschnittsweise auf dem Schweißabschnitt 203 auf oder steht mit diesem in Kontakt.

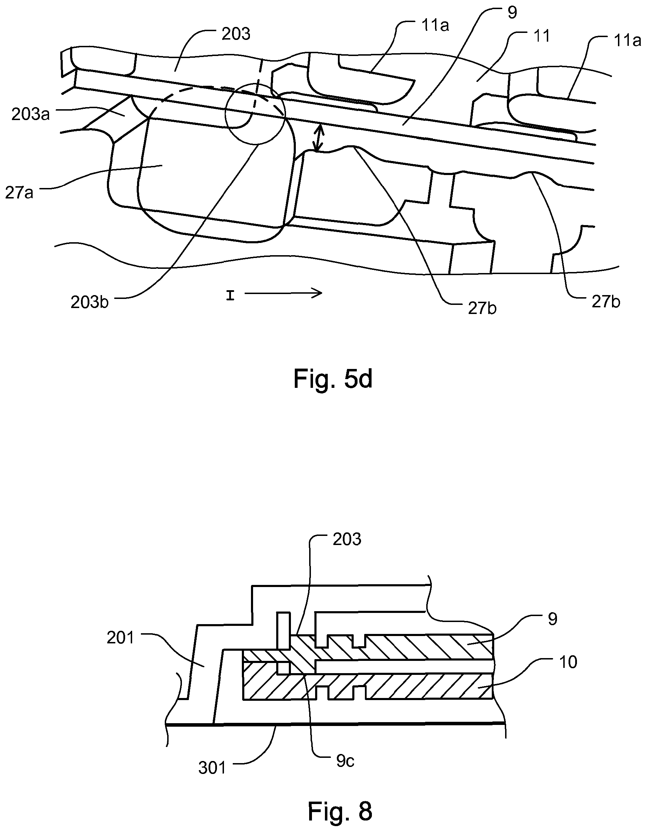

Bevorzugt steht der erste Auflageabschnitt 27a über den Schweißabschnitt 203 in einer Richtung I über, also in Richtung zum zentralen Abschnitt der vorderen Stützstruktur 27 oder der Filtermembran 9. Dies ist in Fig. 5d vergrößert dargestellt und zu dieser weiter erläutert.

Im Beispiel der Fig. 5a weist die vordere Stützstruktur 27 optional Noppen oder Vorsprünge 27b auf, die sich vorzugsweise von jener Seite der vorderen Stützstruktur 27 erheben, an welcher die vordere Stützstruktur 27 der Filtermembran 9 anliegt oder mit welcher sie der Filtermembran 9 zugewandt ist.

Die Noppen oder Vorsprünge 27b sind randseitig und/oder in einem mittleren Bereich der vorderen Stützstruktur 27 angeordnet. Sie weisen Spalten zwischen sich auf, so dass ein Gasaustausch zwischen ihnen hindurch möglich ist.

Im Beispiel der Fig. 5a weist die vordere Stützstruktur 27 eine oder mehrere Noppen oder Vorsprünge 27c auf, die sich vorzugsweise von jener Seite der vorderen Stützstruktur 27 erheben, welcher jener Seite der vorderen Stützstruktur 27, die der Filtermembran 9 zugewandt ist, gegenüberliegt.

Die in Fig. 5a ferner gezeigte Gummimatte 301 drückt auf die Noppen 27c der vorderen Stützstruktur 27.

Die Noppen oder Vorsprünge 27c ragen vorzugsweise um ein vorbestimmtes Maß in einer Richtung D vor. Die Richtung D steht senkrecht zur Haupterstreckungsebene der Filtermembran 9 und/oder senkrecht zur Haupterstreckungsrichtung der vorderen Stützstruktur 27. Das Maß kann derart vorbestimmt sein, dass die Stirnseiten 27c' bis auf die Gummimatte 301 reichen. Das Maß kann derart vorbestimmt sein, dass die Stirnseiten 27c' bis auf ein Niveau N reichen, an welchem der Kassettenkörper 201 in Richtung D mit einer in Fig. 5a nicht dargestellten Folie bedeckt ist.

Im Beispiel der Fig. 5a weist die vordere Stützstruktur 27 optional einen zweiten Auflageabschnitt 27d auf. Der zweite Auflageabschnitt 27d liegt auf einem Stützabschnitt 204 auf. Der Stützabschnitt 204 kann als Vorsprung, Rand oder Kante des Kassettenkörpers 201 ausgestaltet sein.

Der Stützabschnitt 204 und/oder der zweite Auflageabschnitt 27d können umlaufend geschlossen verlaufen, was aufgrund der Schnittdarstellung in Fig. 5c dieser Figur nicht zu entnehmen ist.

Der erste Auflageabschnitt 27a liegt bezogen auf die Richtung I weiter innen als der zweite Auflageabschnitt 27d.

**Fig. 5b** zeigt schematisch sehr vereinfacht und nur in Ausschnitten die Blutkassette 200 einer wiederum weiteren, beispielhaften erfindungsgemäßen Ausführungsform mit dem Kassettenkörper 201, einer Filtermembran 9, einer vorderen Stützstruktur 27 und einer Bördelung oder einer Bördelkante 205. Die Bördelkante 205 dient dem Verklemmen der vorderen Stützstruktur 27 am Kassettenkörper 201 und insbesondere einer Verpressung zwischen dem ersten Auflageabschnitt 27a und dem Schweißabschnitt 203.

**Fig. 5c** zeigt schematisch sehr vereinfacht und nur in Ausschnitten die Blutkassette 200 einer wiederum weiteren, beispielhaften erfindungsgemäßen Ausführungsform mit dem Kassettenkörper 201, einer Filtermembran 9, einer vorderen Stützstruktur 27 und einer Schweißverbindung zwischen dem zweiten Auflageabschnitt 27d der vorderen Stützstruktur 27 und dem Stützabschnitt 204 des Kassettenkörpers 201.

Die zwischen dem zweiten Auflageabschnitt 27d und dem Stützabschnitt 204 bestehende Schweißverbindung kann umlaufend geschlossen verlaufen, was aufgrund der Schnittdarstellung in Fig. 5c dieser Figur jedoch nicht zu entnehmen ist.

**Fig. 5d** zeigt in vergrößerter Darstellung, dass der erste Auflageabschnitt 27a den Schweißabschnitt 203 sowie Teile der Schweißnaht 203a, mit welchem der erste Auflageabschnitt 27a mit dem Schweißabschnitt 203 verbunden ist, kontaktiert oder berührt.

Zu sehen ist ferner, dass der erste Auflageabschnitt 27a einen Abschnitt 203b aufweist, der den Schweißabschnitt 203 in - hier exemplarisch genau - einer Richtung I überragt oder über diesen übersteht. Letzteres ist ein optionales Merkmal, das bereits in Fig. 5a bis Fig. 5c gezeigt ist.

Ein freier Abstand zwischen den Noppen oder Vorsprüngen 27b der vorderen Stützstruktur 27 einerseits und der Filtermembran 9 anderseits soll 0,5 mm, bevorzugt 0,3 mm nicht übersteigen. Dieser Abstand ist in Fig. 5d mittels Doppelpfeil gekennzeichnet. Ist er größer als vorstehend angegeben, so kann die Filtermembran 9 zu stark durchgebogen werden, bevor sie auf den Noppen oder Vorsprüngen 27b der vorderen Stützstruktur 27 zu liegen kommt. Eine unerwünscht hohe mechanische Belastung könnte die Folge sein.

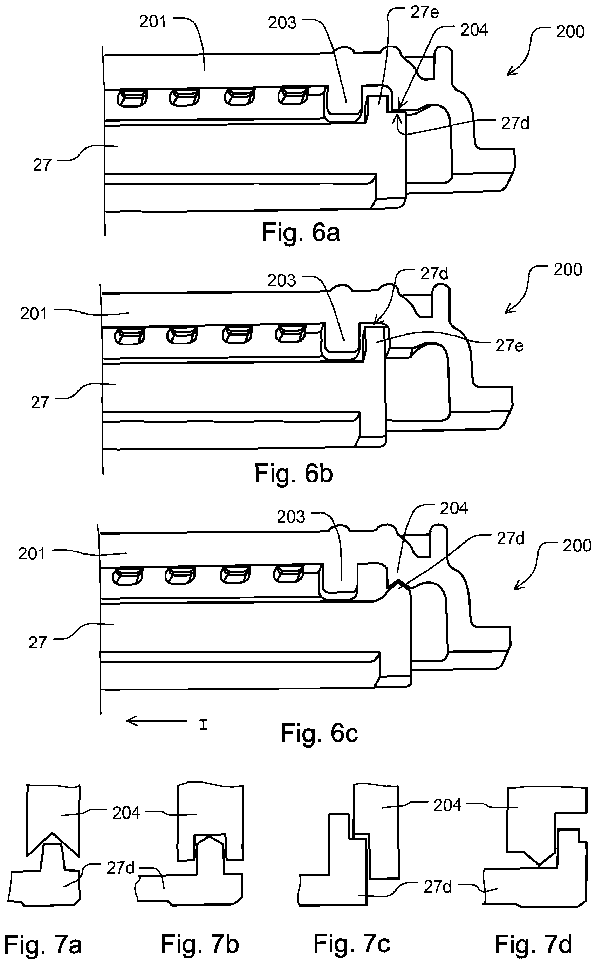

**Fig. 6a** bis **6c** zeigen Abschnitte sowohl des Kassettenkörpers 201 als auch der vorderen Stützstruktur 27 in unterschiedlichen Ausführungsformen der erfindungsgemäßen Blutkassette 200.

Nicht gezeigt ist in Fig. 6a bis 6c die Filtermembran 9, weshalb der Schweißabschnitt 203 in diesen Figuren auch nicht in einem verschweißten Zustand gezeigt sein kann.

Der zweite Auflageabschnitt 27d ist in Fig. 6a als umlaufende Fläche ausgestaltet, deren Ebene zu einer Haupterstreckungsebene der nicht gezeigten Filtermembran 9 oder zur Haupterstreckungsebene der vorderen Stützstruktur 27 - wie im in Fig. 5a gezeigten Beispiel - parallel verläuft.

An einer inneren Seite (links in Fig. 6a, siehe den Pfeil I) erhebt sich ein - vorzugsweise ebenfalls umlaufender - Kragen oder Rand 27e, dessen Innen- und/oder Außenseite im rechten Winkel zur umlaufenden Fläche des zweiten Auflageabschnitts 27d stehen können. Der Rand 27e kann als Zentrierhilfe beim Einführen der vorderen Stützstruktur 27 in den Kassettenkörper 201 verstanden werden. Er kann beim Halten der vorderen Stützstruktur 27, während diese mit dem Kassettenkörper 201 verschweißt wird, dienen.

Obwohl der Rand 27e in Fig. 6a an einer inneren Seite des zweiten Auflageabschnitts 27d liegt, ist erfindungsgemäß auch vorgesehen, ihn alternativ an der äußeren Seite des zweiten Auflageabschnitts 27d vorzusehen.

In Fig. 6b gehen der zweite Auflageabschnitt 27d und der vorstehende Rand 27e ineinander über; der zweite Auflageabschnitt 27d kann als Stirnabschnitt des vorstehenden Rands 27e verstanden werden.

In Fig. 6c sind der zweite Auflageabschnitt 27d und der Stützabschnitt 204 des Kassettenkörpers 201 im in Fig. 6c gezeigten Querschnitt nicht eben, sondern gekrümmt, dreieckig oder mehreckig. Dies kann eine Zentrierwirkung begünstigen. Zudem erhöht sich vorteilhaft die für einen Kontakt zwischen Auflageabschnitt 27d und dem Stützabschnitt 204 z. B. für ein Verschweißen zur Verfügung stehende Fläche (ceteris paribus). Die vorgenannten Vorteile können auch erzielt werden, wenn nur der zweite Auflageabschnitt 27d oder der Stützabschnitt 204 des Kassettenkörpers, nicht aber beide, wie vorstehend beschrieben geformt sind. Beispiele hierfür finden sich in den folgenden Figuren.

Die **Fig. 7a** bis **7d** zeigen verschiedene Ausführungsformen des zweiten Auflageabschnitts 27d und des Stützabschnitts 204 der erfindungsgemäßen Blutkassette 200.

Fig. 7a und Fig. 7b zeigen Ausführungen, welche der Ausgestaltung gemäß Fig. 6c entsprechen oder dieser ähnlich sind.

Fig. 7c zeigt eine Ausführung, welche der Ausgestaltung gemäß Fig. 6a entspricht oder dieser ähnlich ist.

Fig. 7d zeigt eine Ausführung, welche einer Kombination der Ausgestaltungen gemäß Fig. 6b und 6c entspricht.

Die Fig. 7b und 7c zeigen dabei Ausgestaltungen, welche sich angesichts des Schrumpfens beim Schweißen besonders gut eignen.

**Fig. 8** zeigt eine zweite Filtermembran 10, die anstelle oder ergänzend zur Stützstruktur 27 vorgesehen ist.

Die Filtermembran 9, die in einigen, beispielhaften Ausführungsformen zur besseren Unterscheidung auch als erste Filtermembran bezeichnet werden könnte, weist einen Schweißabschnitt oder eine Verschweißung 9c auf, mittels welcher sie mit einer zweiten Filtermembran 10 verbunden ist.

Die zweite Filtermembran 10 kann hinsichtlich der Merkmale, welche zur Filterwirkung beitragen, wie die Filtermembran 9 ausgestaltet sein. Dies ist optional, also nicht zwingend erforderlich.

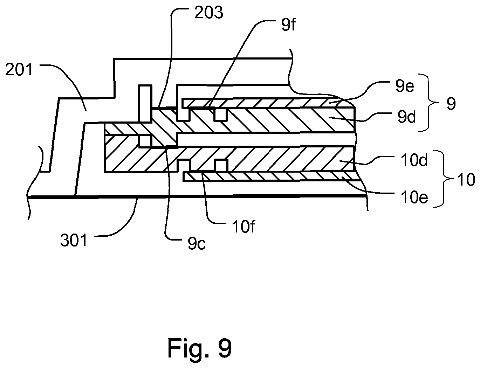

**Fig. 9** zeigt wie Fig. 8 eine zweite Filtermembran 10, die anstelle oder ergänzend zur Stützstruktur 27 vorgesehen ist.

Sowohl die Filtermembran 9 als auch die zweite Filtermembran 10 sind in der Ausführungsform der Fig. 9 in sowohl ein Trägervlies 9d bzw. 10d eine Teflonschicht 9e bzw. 10e unterteilt oder weisen diese jeweils auf.

Dabei sind die Filtermembran 9 und die zweite Filtermembran 10 derart angeordnet, dass jeweils ihr Trägervlies 9d bzw. 10d einander zugewandt sind. Die Teflonschichten 9e bzw. 10e liegen jeweils an jener Seite des Trägervlieses 9d bzw. 10d, welches der jeweils anderen Filtermembran 10 bzw. 9 abgewandt ist. Anders ausgedrückt zeigen die Teflonschichten 9e bzw. 10e somit jeweils nach "außen".

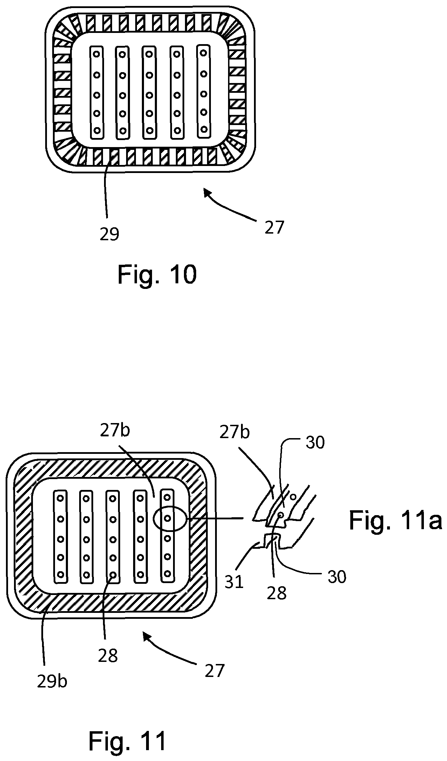

Neben der bereits aus Fig. 8 bekannten Verschweißung 9c der Filtermembran 9 mit der zweiten Filtermembran 10 und dem ebenfalls aus Fig. 8 bekannten Schweißabschnitt 203 sind in Fig. 10 zwei optionale, weitere Schweißnahtabschnitte 9f und 10f gezeigt.

Schweißnahtabschnitt 9f verbindet mittels Verschweißung das Trägervlies 9d mit der Teflonschicht 9e. Schweißnahtabschnitt 10f verbindet mittels Verschweißung das Trägervlies 10d mit der Teflonschicht 10e.

Schweißnahtabschnitt 9f kann wie Schweißnahtabschnitt 10f umlaufend vorgesehen sein.

Die Teflonschicht 9e bzw. 10e kann jeweils aus PTFE (Polytetrafluoroethylen) oder ePTFE (expanded Polytetrafluoroethylen) gefertigt sein oder solches aufweisen.

**Fig. 10** zeigt eine weitere Ausführungsform der vorderen Stützstruktur 27. Diese vordere Stützstruktur 27 weist in ihrem dem Schweißabschnitt 203 zugewandten Bereich eine profilierte Struktur 29 auf. Die Profilierung 29 kann dabei senkrecht oder annährend senkrecht ins Innere der Stützstruktur 27 gerichtet sein oder verlaufen, so, dass zwischen den erhabenen Profilen Kanäle entstehen. Diese Profilierung 29 (Kombination zwischen erhabenen Strukturen einerseits und Kanälen andererseits) kann bewirken, dass bei einem Aufschmelzen der Struktur zum Befestigen der Filtermembran an der vorderen Stützstruktur 27 das Material nicht oder nur wenig in den zentralen Bereich der Filtermembran eindringt, sondern sich überwiegend in die Kanalbereiche ausdehnt.

Dies ist schematisch in **Fig. 11** gezeigt, wo sich das Material der erhabenen Strukturen (schraffierter Bereich der Fig. 10) in den Bereich der Kanäle hin oder in diese hinein erstreckt. Die Ausführungsform der Fig. 11 der vorderen Stützstruktur 27 kann also durch ein wenigstens teilweises Aufschmelzen der erhabenen Bereiche der vorderen Stützstruktur 27 der Fig. 10 herstellbar sein. Dabei kann bei der aufgeschmolzenen Profilierung 29b die Profilierung 29 wenigstens teilweise erkennbar sein. Die vordere Stützstruktur 27 kann, abweichend von der Darstellung der Fig. 11, auch als Parallelogramm ausgebildet sein.

Die Profilierung 29 kann umlaufend in der vorderen Stützstruktur 27 vorgesehen sein. Diese vordere Stützstruktur 27 kann einstückig ausgebildet sein und aus einem Material bestehen. Es hat sich gezeigt, dass diese Ausführungsform geeignet sein kann, auch ohne zusätzliche weichere Stützelemente die Strahlen- oder ebeamsterilisierte Filtermembran so zu stützen, dass eine Delamination der Filtermembran vermieden werden kann.

Die folgenden Merkmale werden zwar anhand der Ausführungsform der **Fig. 11** beschrieben, können jedoch auch bei allen anderen Ausführungsformen realisiert sein. Die vordere Stützstruktur 27 kann Öffnungen 28 aufweisen. Diese können in Vertiefungen 30 angeordnet sein, so dass zwischen den Öffnungen 28 auch flächig Noppen oder Vorsprünge 27b ausgebildet sein können. Im Bereich der Öffnungen 28 kann die vordere Stützstruktur 27 ferner dünner ausgebildet sein, als in anderen Bereichen. Das Prinzip ist in dem eingekreisten Bereich in einem Schnitt in Schrägansicht von vorne in Fig. 11a dargestellt. Dadurch kann die vordere Stützstruktur 27 zusätzlich zu den flächigen Noppen oder Vorsprüngen 27b, die der Filtermembran zugewandt sind, auf der der Filtermembran abgewandten Seite eine Profilierung 31 aufweisen. Die dünnere Ausgestaltung der Stützstruktur 27 im Bereich der Öffnungen kann einen leichteren Gasdurchtritt (geringerer Flusswiderstand) ermöglichen; die zusätzliche Profilierung 31 kann eine Stabilisierung in Form einer höheren Biegesteifigkeit bewirken.

Alle oben beschriebenen vorderen Stützstrukturen 27 und Blutkassettenkörper können Polypropylen aufweisen oder aus diesem bestehen.

Fig. 11a zeigt in Vergrößerung einen Ausschnitt der Fig. 11.

### Bezugszeichenliste

- 1: Fluidaufnahmekammer
- 3: Fluidzuführungskammer
- 5: medizinisches Fluid
- 7: Fluidspiegel oder -pegel
- 9: Filtermembran
- 9a: Rückseite der Filtermembran
- 9b: Vorderseite der Filtermembran
- 9c: Verschweißung mit zweiter Filtermembran
- 9d: Trägervlies
- 9e: Teflonschicht
- 9f: Schweißnahtabschnitt
- 10: zweite Filtermembran
- 10d: Trägervlies
- 10e: Teflonschicht
- 10f: Schweißnahtabschnitt
- 11: hintere Stützstruktur
- 11a: Noppen oder Vorsprünge
- 13: Drainagestruktur
- 15: Befestigungseinrichtungen
- 17: Fluidkonnektor
- 19: zweites, gasförmiges Fluid
- 21: unterer Bereich
- 23: oberer Bereich
- 27: vordere Stützstruktur
- 27a: erster Auflageabschnitt der vorderen Stützstruktur
- 27b: Noppen oder Vorsprünge
- 27c: Noppen oder Vorsprünge
- 27c': Stirnseiten
- 27d: zweiter Auflageabschnitt
- 27e: Rand, Kragen
- 28: Öffnungen
- 29: Profilierung
- 29b: aufgeschmolzene Profilierung
- 30: Vertiefungen
- 31: Profilierung
- 33: Nachgiebigkeitsstruktur
- 35: Fluidanschluss
- 37: erste Ringzone bzw. erster Ringbereich
- 39: zweite Ringzone bzw. zweiter Ringbereich

- 100: Filtereinrichtung
- 200: Blutkassette als Beispiel einer medizinischen Einrichtung
- 201: Kassettenkörper oder Kassettengrundkörper, Hartteil, Einrichtungskörper
- 203: Schweißabschnitt
- 203a: Schweißnaht
- 203b: Abschnitt
- 204: Stützabschnitt
- 205: Bördelung oder Bördelkante
- 229: Abdeckungselement, Folie
- 300: Blutbehandlungsvorrichtung
- 301: Gummimatte
- D: Richtung
- I: Richtung
- N: Niveau

## Patentansprüche

1. Medizinische Einrichtung (200) mit
- einem Fluidsystem für ein erstes medizinisches Fluid (5), insbesondere Blut;
- einem Einrichtungskörper (201); und
- wenigstens einer hydrophoben Filtereinrichtung (100) mit einer Filteroberfläche, durch welche dem Fluidsystem wenigstens ein zweites, gasförmiges Fluid (19), insbesondere Luft, zuführbar ist,
wobei die Filtereinrichtung 100 wenigstens eine Filtermembran (9) aufweist;
wobei die Filtermembran (9) eine Rückseite (9a) und eine Vorderseite (9b) hat;
wobei die Filtermembran (9) mit ihrer Rückseite (9a) mit einem Abschnitt des Einrichtungskörpers (201) entlang eines Schweißabschnitts (203) verschweißt ist;
wobei die Filtereinrichtung (100) an der Vorderseite (9b) der Filtermembran (9) eine vordere Stützstruktur (27) aufweist;
**dadurch gekennzeichnet, dass**
die vordere Stützstruktur (27) angeordnet ist, um mittels eines ersten Auflageabschnitts (27a) der vorderen Stützstruktur (27) mit dem Schweißabschnitt (203) in Kontakt zu stehen,
**und dass**
der erste Auflageabschnitt (27a) der vorderen Stützstruktur (27) den Schweißabschnitt (203) des Einrichtungskörpers (201) in wenigstens einer Richtung (I) oder auf einer Seite des Schweißabschnitts (203) überragt oder über diesen übersteht in Richtung auf einen inneren oder zentralen Abschnitt der Filtermembran (9) oder der vorderen Stützstruktur (27) und/oder in Richtung eines Bereichs der Filtermembran (27), der im Gebrauch mit Druck oder am stärksten mit Druck beaufschlagt wird.

2. Einrichtung nach Anspruch 1, wobei die vordere Stützstruktur (27) einen zweiten Auflageabschnitt (27d) aufweist, wobei die vordere Stützstruktur (27) angeordnet ist, um mittels eines zweiten Auflageabschnitts (27d) mit einem Stützabschnitt (204) des Einrichtungskörpers (201) in Kontakt zu stehen.

3. Einrichtung nach Anspruch 1 oder 2 wobei der zweite Auflageabschnitt (27d) mit dem Stützabschnitt (204) des Einrichtungskörpers (201) verschweißt ist.

4. Einrichtung nach Anspruch 3, wobei der Schweißabschnitt (203) und/oder der erste Auflageabschnitt (27a) umlaufende oder geschlossene Abschnitte sind.

5. Einrichtung nach Anspruch 3 oder 4, wobei der zweite Auflageabschnitt (27d) mit dem Stützabschnitt (204) des Einrichtungskörpers (201) verklemmt ist.

6. Einrichtung nach einem der vorangegangenen Ansprüche, wobei der erste Auflageabschnitt (27a) der vorderen Stützstruktur (27) aus einem weicheren Material ist als andere Abschnitte oder als alle anderen Abschnitte der vorderen Stützstruktur (27).

7. Einrichtung nach einem der vorangegangenen Ansprüche, wobei die vordere Stützstruktur (27) aus genau einem Material gefertigt ist oder aus genau einem Material besteht.

8. Einrichtung nach einem der vorangegangenen Ansprüche, wobei der erste Auflageabschnitt (27a) der vorderen Stützstruktur (27) mit dem Schweißnahtabschnitt (203) des Einrichtungskörpers (201) in Kontakt steht.

9. Einrichtung nach einem der vorangegangenen Ansprüche, wobei der erste Auflageabschnitt (27a) der vorderen Stützstruktur (27) lösbar von weiteren Abschnitten der vorderen Stützstruktur (27) mit letzteren verbunden ist.

10. Einrichtung nach einem der vorangegangenen Ansprüche, wobei sich der erste Auflageabschnitt (27a) der vorderen Stützstruktur (27) farblich von weiteren Abschnitten der vorderen Stützstruktur (27) unterscheidet.

11. Einrichtung nach einem der vorangegangenen Ansprüche, wobei die vordere Stützstruktur (27) Öffnungen (28) aufweist, deren Durchmesser oder Breite maximal 0,7 mm, vorzugsweise maximal 0,5 mm beträgt.

12. Einrichtung nach einem der vorangegangenen Ansprüche, wobei die vordere Stützstruktur (27) eine zweite Filtermembran (10) ist oder aufweist.

13. Einrichtung nach einem der vorangegangenen Ansprüche, wobei die medizinische Einrichtung (200) eine Blutkassette ist.

14. Einrichtung nach einem der vorangegangenen Ansprüche, wobei der erste Auflageabschnitt (27a) der vorderen Stützstruktur (27) ein während der Herstellung der Einrichtung durch Aufschmelzen oder vorübergehendem Verflüssigen von Material entstandener Abschnitt ist oder einen solchen aufweist.

## Claims

1. A medical device (200) comprising:
- a fluid system for a first medical fluid (5), in particular blood;
- a device body (201); and
- at least one hydrophobic filter device (100) with a filter surface through which at least a second gaseous fluid (19), in particular air, may be supplied to the fluid system,
wherein the filter device (100) comprises at least one filter membrane (9);
wherein the filter membrane (9) has a rear side (9a) and a front side (9b);
wherein the filter membrane (9) is welded with its rear side (9a) to a section of the device body (201) along a weld section (203);
wherein the filter device (100) comprises a front support structure (27) at the front side (9b) of the filter membrane (9);
**characterized in that**
the front support structure (27) is arranged to be in contact with the weld section (203) through a first contact section (27a) of the front support structure (27),
**and in that**
the first contact section (27a) of the front support structure (27) overhangs the weld portion (203) of the device body (201) in at least one direction (I) or on one side of the weld portion (203) or protrudes over it towards an inner or central section of the filter membrane (9) or of the front support structure (27), and/or towards an area of the filter membrane (9) which is pressurized or most pressurized in use.

2. The device according to claim 1, wherein the front support structure (27) comprises a second contact section (27d), the front support structure (27) being arranged to contact a support section (204) of the device body (201) through a second contact section (27d).

3. The device according to claim 1 or 2, wherein the second contact section (27d) is welded to the support section (204) of the device body (201).

4. The device according to claim 3, wherein the weld section (203) and/or the first contact section (27a) is/are circumferential or closed sections.

5. The device according to claim 3 or 4, wherein the second contact section (27d) is clamped to the support section (204) of the device body (201).

6. The device according to anyone of the preceding claims, wherein the first contact section (27a) of the front support structure (27) is made of a softer material than other sections or than all other sections of the front support structure (27).

7. The device according to anyone of the preceding claims, wherein the front support structure (27) is made of exactly one material, or consists of exactly one material.

8. The device according to anyone of the preceding claims, wherein the first contact section (27a) of the front support structure (27) contacts the weld section (203) of the device body (201).

9. The device according to anyone of the preceding claims, wherein the first contact section (27a) of the front support structure (27) is connected to the latter from further sections of the front support structure (27) in a releasable manner.

10. The device according to anyone of the preceding claims, wherein the first contact section (27a) of the front support structure (27) differs in color from further sections of the front support structure (27).

11. The device according to anyone of the preceding claims, wherein the front support structure (27) comprises openings (28) whose diameter or width is at most 0.7 mm, preferably at most 0.5 mm.

12. The device according to anyone of the preceding claims, wherein the front support structure (27) is or comprises a second filter membrane (10).

13. The device according to anyone of the preceding claims, wherein the medical device (200) is a blood cassette.

14. The device according to anyone of the preceding claims, wherein the first contact section (27a) of the front support structure (27) is or comprises a section obtained by melting or temporarily liquefying material during the manufacturing of the device.

## Revendications

1. Un dispositif médical (200) comprenant:
- un système fluidique pour un premier fluide médical (5), notamment du sang;
- un corps de dispositif (201); et
- au moins un dispositif de filtration hydrophobe (100) doté d'une surface de filtration au travers de laquelle au moins un second fluide gazeux (19), notamment de l'air, peut être amené au système fluidique,
où le dispositif de filtration (100) comprend au moins une membrane de filtration (9);
où la membrane de filtration (9) a une face arrière (9a) ainsi qu'une face avant (9b);
où la membrane de filtration (9) est soudée par sa face arrière (9a) à une partie du corps de dispositif (201) le long d'une partie soudée (203);
où le dispositif de filtration (100) comprend une structure de support avant (27) sur la face avant (9b) de la membrane de filtration (9);
**caractérisé en ce que**
la structure de support avant (27) est agencée de façon à être en contact avec la partie soudée (203) au moyen d'une première section de contact (27a) de la structure de support avant (27),
**et en ce que**
la première section de contact (27a) de la structure de support avant (27) surplombe ou fait saillie au-delà de la partie soudée (203) du corps de dispositif (201) dans aumoins une direction (I) ou sur une face de la partie soudée (203) vers une partie intérieure ou centrale de la membrane de filtration (9) ou de la structure de support avant (27) et/ou vers une zone de la membrane de filtration (9) qui, en cours d'utilisation, est soumise à une pression ou à la pression la plus forte.

2. Le dispositif selon la première revendication, où la structure de support avant (27) comprend une seconde section de contact (27d), la structure de support avant (27) étant agencée de façon à être en contact avec une partie de support (204) du corps de dispositif (201) au moyen d'une seconde section de contact (27d).

3. Le dispositif selon la revendication 1 ou 2, où la seconde section de contact (27d) est soudée à la partie de support (204) du corps du dispositif (201).

4. Le dispositif selon la revendication 3, où la partie soudée (203) et/ou la première section de contact (27a) est/sont des parties circonférentielles ou fermées.

5. Le dispositif selon la revendication 3 ou 4, où la seconde section de contact (27d) est coincée avec la partie de support (204) du corps du dispositif (201).

6. Le dispositif selon l'une quelconque des revendications précédentes, où la première section de contact (27a) de la structure de support avant (27) est faite d'un matériau plus souple que celui d'autres sections ou que de toutes les autres sections de la structure de support avant (27).

7. Le dispositif selon l'une quelconque des revendications précédentes, où la structure de support avant (27) est fabriquée dans exactement un matériau ou est constituée d'exactement un matériau.

8. Le dispositif selon l'une quelconque des revendications précédentes, où la première section de contact (27a) de la structure de support avant (27) est en contact avec la partie soudée (203) du corps du dispositif (201).

9. Le dispositif selon l'une quelconque des revendications précédentes, où la première section de contact (27a) de la structure de support avant (27) est reliée à cette dernière de manière amovible au départ d'autres sections de la structure de support avant (27).

10. Le dispositif selon l'une quelconque des revendications précédentes, où la première section de contact (27a) de la structure de support avant (27) se différencie en couleur d'autres sections de la structure de support avant (27).

11. Le dispositif selon l'une quelconque des revendications précédentes, où la structure de support avant (27) présente des ouvertures (28) dont le diamètre ou la largeur est d'au plus 0,7 mm, de préférence d'au plus 0,5 mm.

12. Le dispositif selon l'une quelconque des revendications précédentes, où la structure de support avant (27) est ou comprend une seconde membrane de filtration (10).

13. Le dispositif selon l'une quelconque des revendications précédentes, où le dispositif médical (200) est une cassette à sang.

14. Le dispositif selon l'une quelconque des revendications précédentes, où la première section de contact (27a) de la structure de support avant (27) est ou comprend une partie obtenue par fusion ou liquéfaction temporaire de matériau au cours de la fabrication du dispositif.
